# EUROPÄISCHE PATENTSCHRIFT

(11) **EP 2 849 706 B1**
(45) Veröffentlichungstag und Bekanntmachung des Hinweises auf die Patenterteilung: **01.03.2017**
(21) Anmeldenummer: 13719120.1
(22) Anmeldetag: 30.04.2013
(51) Int. Cl.: A61F 13/496, A61F 13/534, A61F 13/535, A61F 13/551, A61F 13/15, A61F 13/49, A61F 13/53

(54) **INKONTINENZARTIKEL IN HÖSCHENFORM**
INCONTINENCE ARTICLE IN THE FORM OF UNDERPANTS
ARTICLE POUR INCONTINENCE EN FORME DE CULOTTE

(30) Priorität: 18.05.2012 DE 102012208395
(43) Veröffentlichungstag der Anmeldung: 25.03.2015
(73) Patentinhaber: Paul Hartmann AG, 89522 Heidenheim (DE)
(72) Erfinder: GASSNER, Oliver, 50678 Köln (DE); BEYRLE, Andreas, 89564 Nattheim (DE); KESSELMEIER, Rüdiger, 89542 Hebrechtingen (DE); MALOWANIEC, Krzysztof, 89522 Heidenheim (DE)
(74) Vertreter: DREISS Patentanwälte PartG mbB
(86) Internationale Anmeldenummer: PCT/EP2013/058979
(87) Internationale Veröffentlichungsnummer: WO 2013/171068

(56) Entgegenhaltungen:
- EP-A1- 1 666 012
- WO-A1-2010/101277
- WO-A1-2013/001825
- JP-A- 2000 024 029
- US-A1- 2003 036 739
- US-A1- 2005 027 272
- US-A1- 2006 218 700
- US-A1- 2012 043 244

## Beschreibung

Die Erfindung betrifft einen gefalteten Inkontinenzartikel in Höschenform für die Aufnahme von Körperausscheidungen mit einem vorderen Bauchabschnitt und einem hinteren Rückenabschnitt, die von separaten und in einer Längsrichtung entlang einer Längsmittelachse voneinander beabstandeten Komponenten gebildet sind, jedoch zur Bildung eines in Quer- oder Hüftumfangsrichtung durchgehenden Bauch- und Rückenbands mit einer in Hüftumfangsrichtung geschlossenen Hüftöffnung an beidseitigen Seitennahtbereichen herstellerseitig miteinander verbunden sind, und mit einem einen Absorptionskörper aufweisenden Schrittabschnitt, der sich in der Längsrichtung zwischen Bauchabschnitt und Rückenabschnitt erstreckt und mit seiner körperabgewandten Seite an den Bauchabschnitt und an den Rückenabschnitt in einem jeweiligen Überlappungsbereich unlösbar angefügt ist, wobei der Bauchabschnitt, der Rückenabschnitt und der Schrittabschnitt gemeinsam Beinöffnungen des Inkontinenzartikels begrenzen, wobei in dem Bauchabschnitt und dem Rückenabschnitt erste Elastifizierungsmittel vorgesehen sind, die sich in einem Abstand voneinander und parallel zueinander in Quer- oder Hüftumfangsrichtung erstrecken und so den Bauchabschnitt und den Rückenabschnitt flächenhaft elastifizieren, wobei in einem schrittseitigen und den Beinöffnungen zugewandten Bereich des Bauchabschnitts und des Rückenabschnitts zweite Elastifizierungsmittel vorgesehen sind, die sich insbesondere ausgehend von den beiden Seitennahtbereichen in Richtung auf eine Längsmittelachse des Inkontinenzartikels erstrecken und sich bis in den Überlappungsbereich von Schrittabschnitt und Bauchabschnitt (im folgenden auch vorderer Überlappungsbereich genannt) bzw. von Schrittabschnitt und Rückenabschnitt (im folgenden auch hinterer Überlappungsbereich genannt) erstrecken, wobei sie dort ihrer elastifizierenden Wirkung benommen sein können, insbesondere geschnitten sein können, wobei der Inkontinenzartikel herstellerseitig in gefaltete Konfiguration gebracht ist, wobei eine erste Falzachse durch eine Quermittelachse gebildet ist.

Es handelt sich also um einen dreikomponentigen Inkontinenzartikel, wobei der Bauchabschnitt, der Rückenabschnitt und der Schrittabschnitt diese drei Komponenten bilden. Der Bauchabschnitt und der Rückenabschnitt sowie der Schrittabschnitt werden als voneinander separate Komponenten einer Herstellungsvorrichtung zugeführt bzw. darin gefördert. Dabei sind die Komponenten typischerweise in einer jeweiligen Förderebene in einem flach oder eben ausgebreiteten Zustand geführt. Dabei werden der Bauchabschnitt und der Rückenabschnitt in der späteren Querrichtung des Inkontinenzartikels gefördert; sie sind dabei in der späteren Längsrichtung des Inkontinenzartikels voneinander beabstandet geführt. Somit verläuft die spätere Quer- oder Hüftumfangsrichtung des Inkontinenzartikels in der Maschinenrichtung der Herstellungsvorrichtung. Der vorgenannte Abstand zwischen Bauchabschnitt und Rückenabschnitt wird dann durch Aufbringen des Schrittabschnitts als dritter Komponente gewissermaßen überbrückt, wobei ein Überlappungsbereich zwischen Schrittabschnitt und Bauchabschnitt und zwischen Schrittabschnitt und Rückenabschnitt gebildet wird, wobei die drei Komponenten in dem jeweiligen Überlappungsbereich unlösbar miteinander gefügt werden. Schließlich werden der Bauchabschnitt und der Rückenabschnitt wie vorstehend erwähnt an beidseitigen Seitennahtbereichen miteinander verbunden. Ein derartiger Inkontinenzartikel ist beispielsweise bekannt aus DE 10 2007 055 628 A1.

Inkontinenzartikel in Höschenform unterscheiden sich prinzipiell von öffenbaren und schließbaren Inkontinenzartikeln in üblicher Windelform dadurch, dass durch die Höschenform der Hüftumfang schon vorgegeben ist und die Anpassung an unterschiedliche Körpergrößen ausgehend von einer Anzahl von Grundgrößen durch eine elastische Dehnbarkeit des Artikels erreicht wird. Hierfür werden in der Regel Elastifizierungsmittel, insbesondere in Form von Bändern oder Fäden, häufig als Lycra-Fäden bezeichnet, in vorgedehntem Zustand (Stretch-Bond-Verfahren) mit Chassismaterialien des Inkontinenzartikels verbunden, das heißt, sie werden in vorgedehntem Zustand an den Chassismaterialien z.B. mittels Kleber fixiert. Infolge ihrer Vorspannung raffen diese Elastifizierungsmittel die Chassismaterialien zusammen und bilden dabei Fältelungen, die typischerweise quer zur Vorspannungsrichtung der Elastifizierungsmittel, hier also in Längsrichtung des Artikels, verlaufen. Der Inkontinenzartikel bzw. die elastifizierten Chassismaterialien des Inkontinenzartikels lassen sich dann wieder elastisch dehnen, wenn der Inkontinenzartikel wie ein Höschen an den Benutzer angelegt wird. Die Chassismaterialien selbst sind hingegen vorzugsweise undehnbar und lassen sich daher in wohldefinierter Weise in der Förderebene in einem flach oder eben ausgebreiteten Zustand führen, so dass die Elastifizierungsmittel dann mit definierter Vorspannung angefügt werden können.

Höschenförmige Inkontinenzartikel der hier in Rede stehenden Art werden typischerweise herstellerseitig gefaltet und in gefalteter Konfiguration, üblicherweise in Folienbeuteln zu wenigstens zehn Stück, an den Handel bzw. den Endverbraucher abgegeben.

Infolge der Höschenform umfasst ein hier in Rede stehender Inkontinenzartikel nach dem Verbinden von Bauchabschnitt und Rückenabschnitt in Seitennahtbereichen bereits eine erste Falzachse, die durch den Scheitelbereich im Schritt des Höschens verläuft. Diese erste Falzachse wird innerhalb der Herstellungsmaschine ausgebildet, sie bildet typischerweise die erste Falzachse des für die Abgabe in den Handel weiter zu faltenden Inkontinenzartikels.

Da man bestrebt ist, beim Falten des Inkontinenzartikels, vorzugsweise im unmittelbaren Anschluss an seine Fertigung und Vereinzelung in einer endlos arbeitenden Herstellungslinie, eine möglichst volumensparende Anordnung zu realisieren, wurde verschiedentlich der Vorschlag unterbreitet, mehrfache Querfaltungen des Absorptionskörpers zu vermeiden, so z.B. EP-A- 1 423 069 B1, JP-A-11-113956. Gemäß EP-A-1 639 908 A1 werden neben der ersten Falzachse im Scheitel des Schrittbereichs zwei weitere in Querrichtung verlaufende Faltungen benötigt, ebenso gemäß WO-A-2011/095908.

Gemäß EP-A-1 140 662 B1 soll eine variierende Dicke bei einem gefalteten Artikel dadurch innerhalb eines Verpackungsbeutels ausgeglichen werden, dass die Artikel in der Stapelung gegensinnig angeordnet werden, ebenso gemäß EP-A-0 780 325 B1. WO 2010/101277 A1, US 2003/0036739 A1, EP 1 666 012 A1, JP 2000-24029 A, US 2006/0218700 A1, US 2005/0027272 A1 und US 2012/0043244 A1 zeigen zum Teil 3-komponentige Inkontinenzartikel in Höschenform mit einer ersten Querfalzache, zwei in Längsrichtung verlaufenden Falzachsen und einer dritten in Querrichtung verlaufenden Falzachse und teilweise einer weiteren Falzachse in Querrichtung.

Der vorliegenden Erfindung liegt die Aufgabe zugrunde, einen höschenförmigen Inkontinenzartikel der eingangs beschriebenen Art so auszubilden, dass er sich in optimaler Weise für die Abgabe in den Handel falten und verpacken lässt, wobei insgesamt eine kompakte und gleichmäßig dicke Form des gefalteten Artikels und hieraus gebildeter stapelförmiger Anordnungen, die dann umverpackt werden, realisiert werden soll.

Diese Aufgabe wird bei einem höschenförmigen Inkontinenzartikel der genannten Art erfindungsgemäß dadurch gelöst, dass beidseits des Absorptionskörpers und außerhalb des Absorptionskörpers je eine zweite im wesentlichen in Längsrichtung erstreckte Falzachse vorgesehen ist, um welche beidseitig seitlich über den Schrittabschnitt vorstehende Bereiche des Bauchabschnitts und Rückenabschnitts in Richtung auf die Längsmittelachse eingeschlagen sind, und dass genau eine dritte in Querrichtung erstreckte Falzachse im Bereich des Absorptionskörpers vorgesehen ist, um die der Artikel gefaltet ist, und dass nach der Faltung um die dritte Falzachse der Hüftrand des in Quer- oder Hüftumfangsrichtung durchgehenden (und seinerseits um die zweiten Falzachsen eingeschlagenen) Bauch- und Rückenbands in Längsrichtung nicht über die erste Falzachse, das heißt nicht über die durch die erste Falzachse gebildete äußere Falzkante des Inkontinenzartikels, übersteht, und dass der Absorptionskörper im Bereich der Quermittelachse ein erstes Flächengewicht an Saugkörpermaterial aufweist und dass ausgehend von der Quermittelachse jeweils entlang einer Längsmittelachse in Richtung auf das bauchabschnittseitige Ende des Absorptionskörpers und in Richtung auf das rückenabschnittseitige Ende des Absorptionskörpers das Flächengewicht an Saugkörpermaterial abnimmt, und dass die dritte Falzachse in einem solchen Abstand in Längsrichtung von der Quermittelachse vorgesehen ist, dass der Absorptionskörper dort ein Flächengewicht an Saugkörpermaterial aufweist, das höchstens 80 % des Werts des ersten Flächengewichts im Bereich der Quermittelachse beträgt.

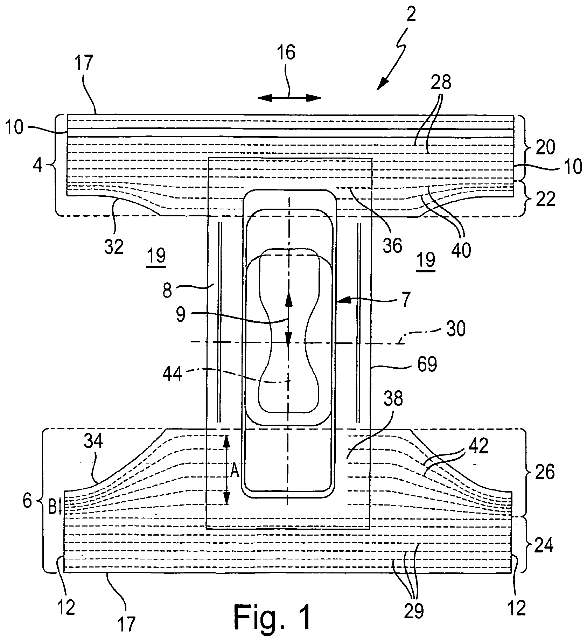

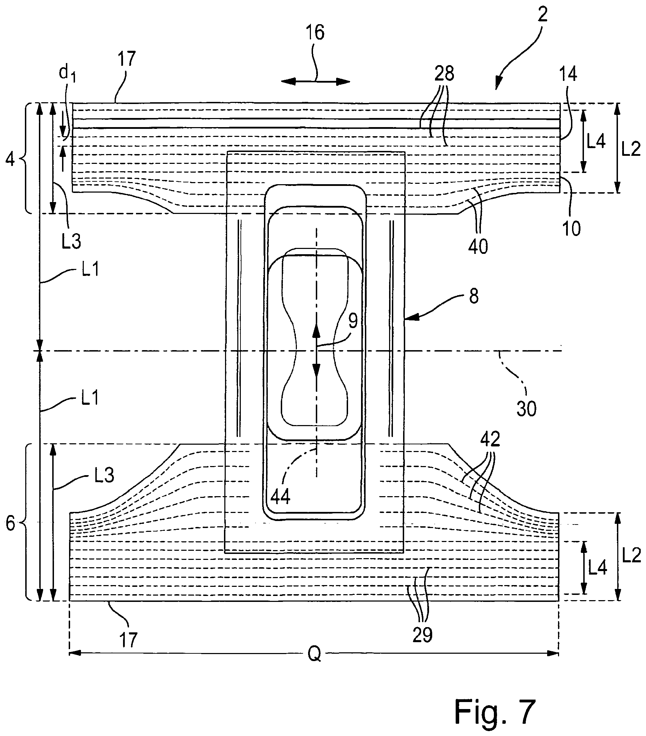

Die Lage der Quermittelachse wird dabei so festgelegt, dass sie die Längserstreckung des Inkontinenzartikels zwischen Hüftrand des Bauchabschnitts und Hüftrand des Rückenabschnitts im ausgedehnten flachgelegten Zustand des Inkontinenzartikels (gemäß Figuren 1 bzw. 7) halbiert. Diese halbe Längserstreckung wird im Folgenden mit L1 bezeichnet. Auch alle übrigen hier erwähnten Abmessungen und Verhältnisse von Abmessungen sind auf diesen in Figur 1 dargestellten flach ausgedehnten Zustand des Inkontinenzartikels und seiner Flachmaterialien bezogen.

Zur Bestimmung des Flächengewichts wird bezüglich der Quermittelachse und der Längsmittelachse ein 25 mm x 25 mm großer Flächenbereich zentriert, in dem das Flächengewicht betrachtet wird. Zur messtechnischen Erfassung des Flächengewichts kann in Dickenrichtung aus dem Absorptionskörper ein 25 mm x 25 mm großer Prüfling ausgestanzt werden. Hierbei werden alle Schichten des Absorptionskörpers zwischen Topsheet und Backsheet berücksichtigt. Zur Bestimmung des Flächengewichts wird der Prüfling zunächst 24 h bei 105°C in einem Trockenschrank getrocknet. Nach dem Abkühlen auf Raumtemperatur wird der Prüfling auf einer Präzisionswaage mit zwei Dezimalen Genauigkeit gewogen.

Es wird also erfindungsgemäß vorgeschlagen, den Absorptionskörper mit einer wie vorstehend beanspruchten Variation seines Flächengewichts an Saugkörpermaterialien auszubilden, also eine Topographie hinsichtlich der Menge und damit hinsichtlich des Flächengewichts des Saugkörpermaterials in der beanspruchten Weise zu realisieren. Zudem wird vorgeschlagen, die dritte in Querrichtung verlaufende Falzachse, bei der es sich neben der ersten Falzachse um die einzige in Querrichtung verlaufende Falzachse des Inkontinenzartikels handelt, derart vorzusehen, dass sie durch den Absorptionskörper verläuft. Jedoch wird erfindungsgemäß vorgeschlagen, dass diese dritte Falzachse durch einen Bereich des Absorptionskörpers verläuft, in dem das Flächengewicht reduziert ist. Auf diese Weise kann erreicht werden, dass der gefaltete Inkontinenzartikel keine wesentlichen und störenden Dickenunterschiede in seiner gefalteten Konfiguration aufweist. Des Weiteren kann erfindungsgemäß erreicht werden, dass das durchgehend elastifizierte Hüft- oder Rückenband nicht in störender Weise über die äußere erste Falzkante des Inkontinenzartikels übersteht. Es kann sich indessen in vorteilhafter Weise in Draufsicht auf den gefalteten Inkontinenzartikel im Wesentlichen wenigstens nahezu bis zu dieser äußeren Falzkante erstrecken. Daher ist es auch nicht erforderlich, dass die gefalteten höschenförmigen Inkontinenzartikel in dem Verpackungsbeutel gegensinnig orientiert werden müssen. Des Weiteren erweist es sich als vorteilhaft, dass sich die einzelnen gefalteten Inkontinenzartikel im Bereich der durch die dritte in Querrichtung verlaufende Falzachse gebildeten äußeren Kante als griffiger erweisen, also insbesondere auch leichter aus dem Verpackungsbeutel entnommen werden können, da der Endverbraucher mit seinen Fingern (Daumen oben und übrige Finger unten) jeweils auf die Außenseite von Chassismaterialien zugreift, die in diesem Bereich mit dem darunter liegenden den Absorptionskörper umfassenden Schrittabschnitt fixiert sind, also nicht weggleiten können.

Die Einfaltung der seitlich überstehenden Bereiche des Bauch- und Rückenbands um die jeweilige zweite Falzachse erfolgt dabei vorzugsweise auf die Bauchseite des Inkontinenzartikels. Dies erweist sich insoweit als vorteilhaft, als der Rückenabschnitt regelmäßig in der Längsrichtung ausladender erstreckt ist und somit beim Einfalten die flächenmäßig ausladenderen Bereiche des Rückenabschnitts die weniger ausladenden Bereiche des Bauchabschnitts überfangen. Hierdurch wird einerseits eine optisch ansprechende Faltung erreicht und andererseits wird verhindert, dass eine Vielzahl von Materialenden von außen wahrnehmbar sind, wodurch auch der weitere Verpackungsvorgang störungsanfälliger würde.

Es erweist sich weiter als vorteilhaft, wenn das Flächengewicht an Saugkörpermaterial im Bereich der dritten Falzachse höchstens 70 %, insbesondere höchstens 60 %, insbesondere höchstens 50 %, insbesondere wenigstens 20 %, insbesondere wenigstens 30 % des Werts des ersten Flächengewichts beträgt.

Weiter erweist es sich als ganz besonders vorteilhaft, wenn das Flächengewicht an Saugkörpermaterial ausgehend von der Quermittelachse entlang einer Längsmittelachse in Richtung auf das bauchabschnittseitige Ende des Absorptionskörpers und/oder in Richtung auf das rückenabschnittseitige Ende des Absorptionskörpers stufenförmig abnimmt, so dass abgestufte Plateaus gebildet werden. Es sei an dieser Stelle darauf hingewiesen, dass die Variation des Flächengewichts an Saugkörpermaterialien nicht zwingend einer dementsprechenden größenmäßigen Variation der topographischen dreidimensionalen Form oder Gestalt, also der jeweiligen Dicke des Absorptionskörpers, entsprechen muss. Typischerweise werden die Saugkörpermaterialien nämlich nach der insbesondere mehrstufigen Ablage innerhalb der Herstellungsmaschine durch Kalanderwalzen komprimiert und kompaktiert. Dennoch bestimmt das Flächengewicht an Saugkörpermaterial des Absorptionskörpers sehr wesentlich das Verhalten des Inkontinenzartikels sowohl beim Falten als auch in seiner anschließenden gefalteten Konfiguration.

Wenn das Flächengewicht stufenförmig abnimmt, so sind die an eine Stufe angrenzenden Bereiche in der Draufsicht auf den eben ausgebreiteten Zustand des Absorptionskörpers (wie in Figur 1 dargestellt) jedenfalls als in der Zeichnungsebene flächenhaft erstreckte Flächen oder Plateaus ersichtlich oder darstellbar. Diese Flächen oder Plateaus müssen nicht zwingend gleichförmiges Flächengewicht an Saugkörpermaterial aufweisen. Sie können von einer Abstufung zur nächsten Abstufung auch mehr oder weniger stark "geneigt" sein, also in Richtung auf die Enden des Saugkörpers abnehmendes Flächengewicht aufweisen. Indessen wird einer Saugkörpertopographie der Vorzug gegeben, bei der das Flächengewicht zwischen stufenförmigen Abnahmen in Richtung der Längsmittelachse betrachtet im Wesentlichen gleichförmig ist.

In weiterer Konkretisierung des Gedankens der stufenförmigen Abnahme des Flächengewichts an Saugkörpermaterial wird vorgeschlagen, dass die Plateaus durch gerade und in Querrichtung verlaufende stufenförmige Übergänge begrenzt sind.

Des Weiteren wird vorgeschlagen, dass der Absorptionskörper gerade und in Längsrichtung erstreckte Längsränder aufweist. Dies bedeutet, dass der Absorptionskörper nach einer bevorzugten Ausführungsform der vorliegenden Erfindung in der Draufsicht auf den eben ausgebreiteten Zustand (Figur 1) die Form eines rechteckförmigen Streifens aufweist. Dieser Streifen ist in Querrichtung vorzugsweise deutlich schmäler als die Breite des Schrittabschnitts, so dass außerhalb des Absorptionskörpers ausreichend Raum für aufstehende Bündchenelemente (Cuffelemente) und Beinelastifizierungsmittel verbleibt. Weiter sind gerade Längsränder vorteilhaft, da solchenfalls keine seitlichen Ohren des Absorptionskörpers seitlich vorstehen, welche die Längsfaltung um die zweiten Falzachsen behindern könnten.

Des Weiteren wird vorgeschlagen, den Absorptionskörper so auszubilden, dass er in seiner bauchabschnittseitigen Hälfte und/oder in seiner rückenabschnittseitigen Hälfte mehrere Plateaus aufweist, wobei deren Flächengewicht an Saugkörpermaterial ausgehend von der Quermittelachse jeweils entlang einer Längsmittelachse in Richtung auf das bauchabschnittseitige Ende des Absorptionskörpers und in Richtung auf das rückenabschnittseitige Ende des Absorptionskörpers von Plateau zu Plateau abnimmt. Eine derartige Ausbildung eröffnet die Möglichkeit, dass der Absorptionskörper in Dickenrichtung mehrschichtig, also durch diskrete Lagen, aufgebaut werden kann. Insofern erweist es sich als besonders vorteilhaft, wenn der Absorptionskörper eine erste Grundschicht und darauf eine zweite durch Flächengewichtsvariation über ihre Erstreckung dreidimensional topologisierte Saugkörperschicht und vorzugsweise darauf eine, insbesondere sanduhrförmige körperzugewandte Flüssigkeitsaufnahme- und -verteilerschicht aufweist. Die erwähnte Grundschicht kann dabei in vorteilhafter Weise über ihre Erstreckung gleichförmiges Flächengewicht aufweisen. Die darüber angeordnete Saugkörperschicht ist gegenüber der Grundschicht in der Längsrichtung und vorzugsweise auch in der Querrichtung zurückgesetzt. Die Zurücksetzung ist typischerweise auf der bauchabschnittseitigen Hälfte des Inkontinenzartikels größer als auf der rückenabschnittseitigen Hälfte. Sie beträgt auf der bauchabschnittseitigen Hälfte in Längsrichtung insbesondere zwischen 10 und 50 mm, insbesondere zwischen 20 und 40 mm, insbesondere zwischen 25 und 40 mm und in der rückenabschnittseitigen Hälfte in Längsrichtung insbesondere zwischen 5 und 20 mm, insbesondere zwischen 5 und 15 mm.

Nach einem weiteren Erfindungsgedanken von besonderer Bedeutung erweist es sich als vorteilhaft, wenn das erste Flächengewicht des Absorptionskörpers ausgehend von der Quermittelachse jeweils entlang der Längsmittelachse in Richtung auf das bauchabschnittseitige Ende des Absorptionskörpers und in Richtung auf das rückenabschnittseitige Ende des Absorptionskörpers im wesentlichen konstant bleibt über eine Erstreckung von wenigstens 20 %, insbesondere von wenigstens 30 %, insbesondere von höchstens 70 % und weiter insbesondere von höchstens 60 % des Abstands der Quermittelachse vom bauchabschnittseitigen Ende des Absorptionskörpers bzw. vom rückenabschnittseitigen Ende des Absorptionskörpers. Hierbei bedeutet das Merkmal "im Wesentlichen konstant", dass die Variation oder Abweichung vom Mittelwert (maximales Flächengewicht minus minimales Flächengewicht) höchstens 5 % betragen soll.

Des Weiteren erweist es sich als vorteilhaft, wenn die Längserstreckung eines Plateaus, welches sich an eine Abstufung im bauchabschnittseitigen Teil und/oder im rückenabschnittseitigen Teil des Absorptionskörpers in Längsrichtung anschließt und durch welches sich die dritte Falzachse erstreckt, wenigstens 15 %, insbesondere wenigstens 20 %, insbesondere höchstens 50 %, insbesondere höchstens 40 %, insbesondere höchstens 30 % des Abstands der Quermittelachse vom bauchabschnittseitigen Ende des Absorptionskörpers bzw. vom rückenabschnittseitigen Ende des Absorptionskörpers beträgt.

Der Absorptionskörper kann ausgehend von der Längsmittelachse in Querrichtung abnehmendes Flächengewicht an Saugkörpermaterial aufweisen. Vorzugsweise nimmt das Flächengewicht an Saugkörpermaterial in Querrichtung nicht zu.

Es erweist sich als vorteilhaft, wenn die dritte Falzachse im Bauchabschnitt und im Rückenabschnitt durch einen jeweiligen Endabschnitt des Absorptionskörpers verläuft, wobei ein jeweiliger Endabschnitt höchstens 1/5, insbesondere höchstens 1/6 und weiter insbesondere höchstens 1/7 der Längserstreckung des Absorptionskörpers erfasst.

Weiter erweist es sich als vorteilhaft, wenn die dritte Falzachse im Überlappungsbereich von Schrittabschnitt und Rückenabschnitt und/oder im Überlappungsbereich von Schrittabschnitt und Bauchabschnitt verläuft. Hierdurch wird erreicht, dass der betreffende Bauch- oder Rückenabschnitt den Bereich der äußeren Falzkante des Inkontinenzartikels im Bereich der dritten Falzachse vollständig überfängt, was optisch und haptisch vorteilhaft ist.

Bei einer bevorzugten Ausführungsform des erfindungsgemäßen gefalteten Inkontinenzartikels erweist es sich als vorteilhaft, dass die Dicke gemessen unter einem Prüfdruck von 20 g/cm² an drei verschiedenen Stellen, nämlich in einem 10 mm von dem der ersten Falzachse zugeordneten Rand beabstandeten Bereich, und in einem 10 mm von dem der dritten Falzachse zugeordneten Rand beabstandeten Bereich und in einem in Längsrichtung dazwischen liegenden Bereich, jeweils um weniger als 6 %, insbesondere um weniger als 5 %, insbesondere um weniger als 4 %, insbesondere um weniger als 3 % von einem arithmetischen Mittelwert der Messungen an den drei Stellen abweicht. Zur Bestimmung der Dicke des gefalteten Inkontinenzartikels werden aus dem gesamten gefalteten Inkontinenzartikel Prüflinge ausgestanzt, die eine Längserstreckung von 50 mm aufweisen und über die gesamte Querrichtung des gefalteten Artikels erstreckt sind. Diese Prüflinge werden in einer Dickenprüfanordnung gegenüber einem Prüfstempel von 100 x 100 mm zentriert angeordnet und mit einem Prüfdruck von 20 g/m² belastet. Obschon die Dicke nicht sehr stark von der Dauer der Druckprüfung abhängt, wird die Dicke nach 30 Minuten Belastung genommen. Zur Bestimmung der Dickenwerte in jedem der drei Bereiche werden jeweils drei gefaltete Artikel berücksichtigt, wobei das jeweilige arithmetische Mittel der Messwerte zugrunde gelegt wird. Die Dicke des gefalteten Inkontinenzartikels an den drei Stellen kann 14 bis 25 mm, insbesondere 14 bis 22 mm, insbesondere 14 bis 20 mm, insbesondere 14 bis 18 mm betragen.

Im Hinblick auf eine kompakte Faltung erweist es sich als besonders vorteilhaft, wenn die Erstreckung (L2) der jeweiligen Seitennaht in Längsrichtung 100 - 170 mm beträgt und wenn das Verhältnis (L2/L1) der Erstreckung (L2) der jeweiligen Seitennaht in Längsrichtung zur Erstreckung (L1) des Inkontinenzartikels zwischen Hüftrand und einer Quermittelachse höchstens 0,42, insbesondere höchstens 0,4, insbesondere höchstens 0,39, insbesondere höchstens 0,38 und weiter insbesondere wenigstens 0,20, weiter insbesondere wenigstens 0,25, weiter insbesondere wenigstens 0,30 beträgt. Die Längserstreckung L1 des hier in Rede stehenden Inkontinenzartikels beträgt bei typischen Größen 320 bis 450 mm, insbesondere 330 bis 440 mm und weiter insbesondere 340 bis 430 mm.

In diesem Zusammenhang erweist es sich auch als vorteilhaft, wenn im Bauchabschnitt und im Rückenabschnitt das Verhältnis (L4/L1) des Abstands (L4) des äußersten hüftzugewandten ersten Elastifizierungsmittels in Längsrichtung vom innersten schrittzugewandten ersten Elastifizierungsmittel zur Erstreckung (L1) des Inkontinenzartikels zwischen Hüftrand und der Quermittelachse höchstens 0,3, insbesondere höchstens 0,29 und insbesondere wenigstens 0,12, insbesondere wenigstens 0,15, insbesondere wenigstens 0,18 beträgt.

Weiter erweist es sich als vorteilhaft, wenn im Bauchabschnitt und/oder im Rückenabschnitt das Verhältnis (d₁/L4) des Abstands (d₁) der ersten Elastifizierungsmittel in Längsrichtung voneinander zum Abstand (L4) des äußersten hüftzugewandten ersten Elastifizierungsmittels in Längsrichtung vom innersten schrittzugewandten ersten Elastifizierungsmittel zwischen 0,08 und 0,25, insbesondere zwischen 0,09 und 0,20, insbesondere zwischen 0,10 und 0,18 beträgt. Dabei erweist es sich als vorteilhaft, wenn der Abstand (d₁) der ersten Elastifizierungsmittel in Längsrichtung voneinander wenigstens 8 mm, insbesondere wenigstens 10 mm, insbesondere 10 - 15 mm, insbesondere 11 - 14 mm, weiter insbesondere 12 - 13 mm beträgt.

Als erste und/oder zweite Elastifizierungsmittel werden vorzugsweise faden- oder bandförmige Elastifizierungsmittel, wie Gummi- oder Polyetherpolyurethan- oder Polyesterpolyurethanfäden, vorzugsweise Elastikfäden wie Lycra®- oder Spandex®-Fäden eingesetzt.

Weiter erweist es sich als vorteilhaft, wenn die Fadenstärke der ersten Elastifizierungsmittel wenigstens 1000 dtex, insbesondere wenigstens 1100 dtex, insbesondere wenigstens 1200 dtex, insbesondere 1200 - 1500 dtex, insbesondere 1200 - 1400 dtex beträgt und/oder wenn die Fadenstärke der zweiten Elastifizierungsmittel 500 -1100 dtex, insbesondere 600 - 1000 dtex, insbesondere 700 - 900 dtex beträgt. Dabei ist die Fadenstärke der ersten Elastifizierungsmittel vorzugsweise größer als die Fadenstärke der zweiten Elastifizierungsmittel.

Die Fadenstärke der Elastifizierungsmittel wird in der Einheit dtex angegeben (1 dtex = 1g/10000 m). Die Fadenstärke wird entsprechend den Prüfvorschriften BISFA, The International Bureau for the Standardization of man-made Fibres, Test methods for bare elastane yarns, Ausgabe 1998, Kapitel 5: "Determination of linear density" (Bestimmung der linearen Dichte) bestimmt. Die Fadenstärke oder lineare Dichte wird durch Bestimmung der Masse eines Prüfling einer bekannten Fadenlänge von 1000 mm (abgetrennt unter einer Standardvorspannung von 0,1 ± 0,01 mN/tex) nach einer Konditionierung unter Standardbedingungen (23°C ± 2°C, 50% ± 5% relative Luftfeuchtigkeit) im entspannten Zustand bestimmt.

Die Fadenstärke (in dtex) errechnet sich aus dem Quotienten der Masse (in g) durch die Länge des Abschnitts (in m) multipliziert mit dem Faktor 10000.

Es werden hierfür fünf Abschnitte mit jeweils etwa 1300 mm Länge des fadenförmigen oder bandförmigen Elastifizierungsmittels unter geringstmöglicher Spannung von einer Rolle oder Packung abgeschnitten, und zwar in unregelmäßigen Abständen von wenigstens 2 m. Diese fünf Abschnitte werden spannungslos relaxiert und unter den Standardbedingungen für wenigstens vier Stunden ruhen gelassen. Sodann wird ein Prüfling von 1000 mm ± 1 mm Länge von dem jeweiligen 1300 mm langen Abschnitt abgeschnitten, während der betreffende Abschnitt unter einer Vorspannung von 0,1 mN/tex gehalten wird. Diese abgetrennten Prüflinge von 1000 mm Länge werden auf eine Genauigkeit von ± 1 % ihrer erwarteten Masse gewogen. Für jeden Prüfling wird dessen Fadenstärke durch Multiplikation der jeweiligen Masse mit dem Faktor 10000 in dtex erhalten. Aus den fünf Prüflingen wird der arithmetische Mittelwert berechnet, der als Fadenstärke für die hier in Rede stehenden Zwecke verwendet wird.

Die Vorspannung ist definiert als der Dehnungsgrad eines gedehnten Elastifizierungsmittels gegenüber dem ungedehnten/relaxierten Ausgangszustand des Elastifizierungsmittels im Zustand des Aufbringens und Fixierens der Elastifizierungsmittel in der Herstellungsmaschine. Der Dehnungsgrad errechnet sich also als Verhältnis der gedehnten Länge L' (=Ausgangslänge L + ΔL) zur Ausgangslänge L, also L'/L.

Wie bereits erwähnt, sind die ersten und zweiten Elastifizierungsmittel zur Erzielung einer Rückstellkraft und damit einer flächenhaften Elastifizierung des Bauchabschnitts und des Rückenabschnitts im vorgespannten Zustand gegenüber den Chassismaterialien fixiert (Stretchbondverfahren). Dabei erweist es sich als vorteilhaft, wenn die ersten Elastifizierungsmittel mit einer Vorspannung fixiert sind, welche um den Faktor von wenigstens 1,1, insbesondere wenigstens 1,2, insbesondere wenigstens 1,3 und insbesondere höchstens 2,0, insbesondere höchstens 1,8, insbesondere höchstens 1,6 größer ist als die Vorspannung, mit welcher die zweiten Elastifizierungsmittel fixiert sind. Dabei erweist es sich als vorteilhaft, wenn die ersten Elastifizierungsmittel mit einer Vorspannung von 3 - 8, insbesondere 3 - 7, insbesondere 4 - 7 und weiter insbesondere 4 - 6 fixiert sind und/oder die zweiten Elastifizierungsmittel mit einer Vorspannung von 2 - 5, insbesondere 2,5 - 4,5, insbesondere 2,5 - 4 und weiter insbesondere 3 - 4 fixiert sind.

Nach einem weiteren an sich unabhängigen Erfindungsgedanken erweist es sich als vorteilhaft, wenn der Schrittabschnitt mittels einer Vielzahl von im Überlappungsbereich von Schrittabschnitt und Bauchabschnitt und im Überlappungsbereich von Schrittabschnitt und Rückenabschnitt vorgesehener, in Querrichtung erstreckter, parallel zueinander verlaufender und voneinander durch kleberfreie Streifen beabstandeter Klebestreifen unlösbar mit dem Bauchabschnitt und mit dem Rückenabschnitt verbunden ist, wobei die Klebestreifen im wesentlichen den gesamten jeweiligen Überlappungsbereich erfassen, und wenn die Breite zumindest derjenigen Klebestreifen, die bezüglich optionaler randseitiger Klebestreifen innen liegen, quer zu ihrer Erstreckung wenigstens 1 mm bis höchstens 5 mm beträgt, und wenn die Breite der kleberfreien Streifen quer zu ihrer Erstreckung wenigstens 1 mm bis höchstens 15 mm beträgt. Durch diese Art der Verbindung des Schrittabschnitts mit dem Bauchabschnitt und dem Rückenabschnitt ergibt sich eine in Querrichtung verlaufende Vorzugsrichtung, die die Faltung des Inkontinenzartikels um die dritte in Querrichtung verlaufende Falzachse begünstigt. Dies ist dadurch zu erklären, dass der streifenförmig aufgebrachte Kleber in die dreidimensional porösen zumeist auf Vliesbasis beruhenden Chassismaterialien einzudringen vermag und diesbezüglich zu einer Versteifung und Strukturierung in der Querrichtung führt.

Weiter insbesondere werden entsprechend dem Verlauf der Klebestreifen und der kleberfreien Streifen in Querrichtung visuell und/oder haptisch wahrnehmbare Strukturen auf der äußeren Sichtseite des Inkontinenzartikels im Überlappungsbereich von Schrittabschnitt und Bauchabschnitt und im Überlappungsbereich von Schrittabschnitt und Rückenabschnitt gebildet.

Diese visuell und/oder haptisch wahrnehmbaren Strukturen erweisen sich insbesondere im gefalteten Zustand des Inkontinenzartikels als vorteilhaft, da hierdurch im Bereich um die dritte in Querrichtung verlaufende Falzachse ein griffiger Kantenbereich gebildet wird.

In Weiterbildung dieses Erfindungsgedankens erweist es sich als vorteilhaft, wenn im Überlappungsbereich von Schrittabschnitt und Bauchabschnitt und/oder im Überlappungsbereich von Schrittabschnitt und Rückenabschnitt zwei äußere randseitige Klebestreifen und in Längsrichtung zwischen diesen eine Vielzahl von innenliegenden Klebestreifen vorgesehen sind, wobei die Breite der randseitigen Klebestreifen größer als die Breite der innenliegenden Klebestreifen ist, insbesondere wenigstens das 4-fache, insbesondere wenigstens das 5-fache und weiter insbesondere höchstens das 8-fache, insbesondere höchstens das 7-fache der Breite der innenliegenden Klebestreifen beträgt.

Die chassisbildenden Materialien von Bauchabschnitt und/oder Rückenabschnitt umfassen vorzugsweise Vliesstoffmaterialien, wie Spinnvliese, Kardenvliese oder Through Air bonded Kardenvliese. Besonders bevorzugt umfasst das chassisbildende Material von Bauchabschnitt und/oder Rückenabschnitt ein Spinnvliesmaterial. Die für Bauchabschnitt und/oder Rückenabschnitt eingesetzten Vliesstoffmaterialien haben vorteilhafterweise ein Flächengewicht von 10 - 30 g/m², weiter vorzugsweise von 15 - 25 g/m². Besonders bevorzugt umfassen der Bauchabschnitt und der Rückenabschnitt ein Spinnvlies, insbesondere aus Polypropylen, insbesondere mit einem Flächengewicht von 15 - 25 g/m².

Der Schrittabschnitt umfasst vorteilhafterweise ein flüssigkeitsundurchlässiges Backsheet-Material und ein Vlies-Topsheet-Material. Das Backsheet umfasst insbesondere eine Folie, insbesondere eines Flächengewichts von 8 - 20 g/m², insbesondere von 8 - 16 g/m², insbesondere von 8 - 14 g/m².

Insbesondere umfasst das Backsheet eine im Gebrauch flüssigkeitsdichte aber gleichwohl atmungsaktive, also wasserdampfdurchlässige, insbesondere mikroporöse Folie.

Der Absorptionskörper umfasst Körperflüssigkeiten absorbierende Saugkörpermaterialien wie natürliche oder synthetische Fasern, insbesondere Zellulosefasern, vorzugsweise in Form von Zellstofffluff und/oder intravernetzte Zellulosefasern. Vorzugsweise umfassen die Saugkörpermaterialien außerdem superabsorbierende Materialien (SAP), insbesondere auf Basis oberflächenvernetzter, teilneutralisierter Polyacrylate.

Des weiteren wird Schutz beansprucht für einen Verpackungsbeutel, der mit erfindungsgemäß gefalteten und ausgebildeten Inkontinenzartikeln befüllt ist.

Weitere Merkmale, Einzelheiten und Vorteile der Erfindung ergeben sich aus den beigefügten Patentansprüchen und aus der zeichnerischen Darstellung und nachfolgenden Beschreibung einer bevorzugten Ausführungsform des erfindungsgemäßen Inkontinenzartikels. In der Zeichnung zeigt:
Figur 1 eine Draufsicht auf einen erfindungsgemäßen Inkontinenzartikel, wobei ein Bauchabschnitt, ein Rückenabschnitt und Schrittabschnitt des Inkontinenzartikels noch nicht zur Bildung einer Höschenform verbunden, sondern in flachgelegtem und eben ausgedehntem Zustand dargestellt sind;
Figur 2a,b schematische Schnittansichten des Schrittabschnitts im Bereich der Quermittellinie bzw. im Überlappungsbereich von Schrittabschnitt und Rückenabschnitt;
Figur 3 eine Figur 1 entsprechende Darstellung, wobei die Fixierung des Schrittabschnitts mit dem Bauchabschnitt und dem Rückenabschnitt mittels Klebestreifen erkennbar ist;
Figur 4 eine vergrößerte ausschnittsweise Darstellung im Bereich des Überlappungsbereichs von Schrittabschnitt und Bauchabschnitt bzw. Schrittabschnitt und Rückenabschnitt des Inkontinenzartikels nach Figur 3;
Figur 5 eine schematische Schnittansicht der wesentlichen Einzelkomponenten der Chassismaterialien entlang der Längsmittelachse des Inkontinenzartikels;
Figur 6 eine schematische Ansicht des fertig konfigurierten Inkontinenzartikels
Figur 7 eine Figur 1 entsprechende Darstellung zur Verdeutlichung von Abmessungen;
Figur 8 eine Figur 1 entsprechende Darstellung zur Verdeutlichung des Aufbaus des Absorptionskörpers und der Faltachsen;
Figur 9 eine schematische Längsschnittansicht des Absorptionskörpers entlang der Längsmittelachse;
Figur 10a,b,c drei schematische Ansichten des Inkontinenzartikels zur Verdeutlichung der Faltung und
Figur 11 eine schematische Ansicht des gefalteten Inkontinenzartikels zur Verdeutlichung der Probennahme bei der Dickenbestimmung.

Die Figuren zeigen einen insgesamt mit dem Bezugszeichen 2 bezeichneten Inkontinenzartikel in Höschenform für die Aufnahme fester und flüssiger Körperausscheidungen. Der Inkontinenzartikel 2 ist aus drei weitestgehend unabhängig voneinander fertigbaren Komponenten, nämlich einem vorderen Bauchabschnitt 4, einem hinteren Rückenabschnitt 6 und einem zwischen diesen angeordneten und einen Absorptionskörper 7 aufweisenden Schrittabschnitt 8 gebildet, wobei der Schrittabschnitt 8 in einer Längsrichtung 9 des Inkontinenzartikels 2 erstreckt ist und mit einem wesentlichen Flächenanteil des Bauchabschnitts 4 einerseits und des Rückenabschnitts 6 andererseits überlappt und im Überlappungsbereich auf noch näher zu beschreibende Weise herstellerseitig unlösbar verbunden ist. Wie aus Figur 1 ersichtlich, führt dies zu einer H-förmigen Grundstruktur des Inkontinenzartikels. Die in Figur 1 dargestellten miteinander gefügten Bestandteile werden dann zur Bildung der Höschenform an jeweiligen seitlichen Längsrandabschnitten 10, 12 des Bauchabschnitts 4 und des Rückenabschnitts 6 ebenfalls herstellerseitig miteinander durch übliche Fügeverfahren verbunden, wodurch beidseits Seitennahtbereiche 14 ausgebildet werden. In diesem herstellerseitig gefertigten höschenförmigen Zustand des Inkontinenzartikels 2 erstrecken sich der Bauchabschnitt 4 und der Rückenabschnitt 6 in einer Quer- oder Hüftumfangsrichtung 16 durchgehend und definieren so mit ihrem Hüftrand 17 eine in Hüftumfangsrichtung geschlossene Hüftöffnung 18; ferner begrenzen sie zusammen mit dem Schrittabschnitt 8 Beinöffnungen 19, durch welche hindurch der Benutzer den Inkontinenzartikel wie ein Höschen anlegt.

Der Bauchabschnitt 4 lässt sich in einen hüftseitigen Bereich 20 und in einen schrittseitigen und den Beinöffnungen 19 zugewandten Bereich 22 unterteilen. Eine entsprechende Unterteilung ist im Rückenabschnitt 6 vorgesehen, und zwar ebenfalls in einen hüftseitigen Bereich 24 und einen schrittseitigen und den Beinöffnungen 19 zugewandten Bereich 26.

In dem hüftseitigen Bereich 20 des Bauchabschnitts 4 und in dem hüftseitigen Bereich 24 des Rückenabschnitts 6 sind erste Elastifizierungsmittel 28, 29 vorgesehen, bei denen es sich insbesondere um fadenförmige Elastifizierungsmittel, wie Lycra-Fäden, handeln kann, die in vorgedehntem Zustand, im sogenannten Stretch-Bond-Verfahren, mit den Flachmaterialien (Chassismaterialien) des Bauchabschnitts 4 und des Rückenabschnitts 6 verbunden sind. Diese ersten Elastifizierungsmittel 28, 29 erstrecken sich in Quer- oder Hüftumfangsrichtung 16 von einem Seitennahtbereich 14 zum anderen.

Der schrittseitige und den Beinöffnungen 19 zugewandte Abschnitt 22 und 26 des Bauchabschnitts 4 bzw. des Rückenabschnitts 6 haben eine von der Quer- oder Hüftumfangsrichtung 16 abweichende in Richtung auf eine Quermittelachse 30 des Schrittabschnitts 8 zulaufende Randkontur 32 bzw. 34. Diese Randkontur 32, 34 ist auch in der Darstellung nach Figur 1 bogenförmig und daher zur Begrenzung der Beinöffnungen 19 geeignet.

Durch diesen Verlauf des schrittseitigen und den Beinöffnungen zugewandten Bereichs 22 bzw. 26 wird auch ein verhältnismäßig großer Überlappungsbereich 36, 38 zwischen Schrittabschnitt 8 und Bauchabschnitt 4 bzw. Rückenabschnitt 6 realisiert, der im Hinblick auf eine reißfeste Verbindung von Schrittabschnitt 8 und Bauchabschnitt 4 bzw. Rückenabschnitt 6 wesentlich ist.

Der jeweilige schrittseitige und den Beinöffnungen 19 zugewandte Bereich 22, 26 des Bauchabschnitts 4 bzw. des Rückenabschnitts 6 ist ebenfalls elastifiziert ausgebildet. Dort sind zweite Elastifizierungsmittel 40 bzw. 42 vorgesehen. Die zweiten Elastifizierungsmittel 40, 42 erstrecken sich jeweils ausgehend von den Seitennahtbereichen 14 in Richtung auf eine Längsmittelachse 44 des Inkontinenzartikels. Wie aus Figur 1 zu erkennen ist, fächern die zweiten Elastifizierungsmittel 40, 42 in Richtung auf die Längsmittelachse 44 auf. Dies bedeutet, dass der Abstand zwischen ihnen in Richtung auf die Längsmittelachse 44 zunimmt. Die zweiten Elastifizierungmittel 40, 42 unterqueren den Schrittabschnitt 8. Sie können im Bereich unterhalb des Absorptionskörpers 7 deaktiviert, d.h. ihrer elastifizierenden Wirkung benommen sein.

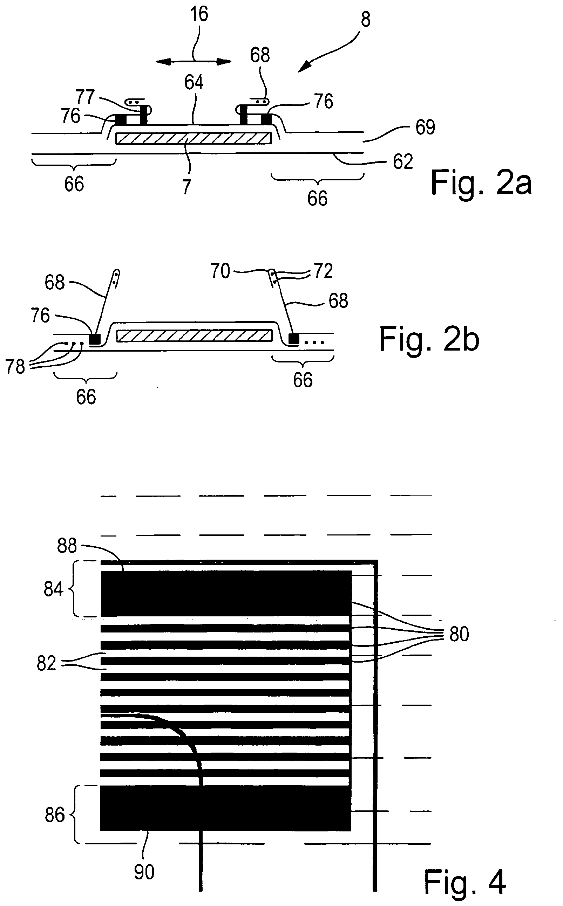

Wie aus Figuren 2a, b ersichtlich umfasst der Schrittabschnitt 8 ein flüssigkeitsundurchlässiges Backsheet-Material 62, das insbesondere von einem atmungsaktiven, jedoch flüssigkeitsdichten Folienmaterial gebildet sein kann, und ein, vorzugsweise auf Vliesbasis beruhendes Topsheet-Material 64. Zwischen dem Backsheet-Material und dem Topsheet-Material ist der Absorptionskörper 7 (nur schematisch dargestellt) angeordnet. Im beispielhaft dargestellten Fall bildet das Backsheet-Material 62 in Querrichtung 16 einen Überhang 66 über den Absorptionskörper 7. Das Topsheet 64 überragt den Absorptionskörper 7 in Querrichtung 16 nur verhältnismäßig geringfügig, und beidseits des Absorptionskörpers 7 ist in Längsrichtung 9 verlaufend jeweils ein aufstehendes Barrieremittel 68 vorgesehen, welches typischerweise als aufstehendes Cuffelement oder Bündchenelement bezeichnet wird und vorzugsweise aus einem hydrophoben, insbesondere flüssigkeitsundurchlässigen Vliesstoffmaterial gebildet ist, welches sich in Querrichtung 16 vorzugsweise bis zu seitlichen Längsrändern 69 des Schrittabschnitts 8 erstreckt. Die distalen Enden 70 der Barrieremittel 68 sind mit weiteren Elastifizierungsmitteln 72 versehen, welche die Barrieremittel 68 im Gebrauch des Inkontinenzartikels gegen die Hautoberfläche des Benutzers anheben. Die seitlichen Barrieremittel 68 sind über schematisch angedeutete Fixierungen 76, 77 auf das Topsheet 64 beziehungsweise auf sich selbst in einer C-förmig gefalteten Konfiguration festgelegt. Außerhalb des Absorptionskörpers 7 also im Bereich des Überhangs 66 sind ferner Beinelastifizierungsmittel 78 vorgesehen, die vorzugsweise mit einem gewissen Abstand vom materialreichen und damit eher biegesteifen Absorptionskörper 7 verlaufen, um einerseits keine zusätzlichen Dehnungs- oder Verwindungskräfte auf den Absorptionskörper auszuüben, was dessen Absorptionsverhalten nachteilig beeinflussen könnte, und um andererseits einen vom Absorptionskörper weitgehend unbeeinflussten flüssigkeitsdichten Beinabschluss zu realisieren. Diese Beinelastifizierungsmittel 78 enden in Längsrichtung 9 mit einem deutlichen Abstand von insbesondere wenigstens 10 mm, vorzugsweise wenigstens 20 mm vor den zweiten Elastifizierungsmitteln 40 und 42 des Bauchabschnitts 4 beziehungsweise des Rückenabschnitts 6. Vorzugsweise enden diese Beinelastifizierungsmittel 78 in Längsrichtung 9 vor dem Bauchabschnitt 4 und dem Rückenabschnitt 6.

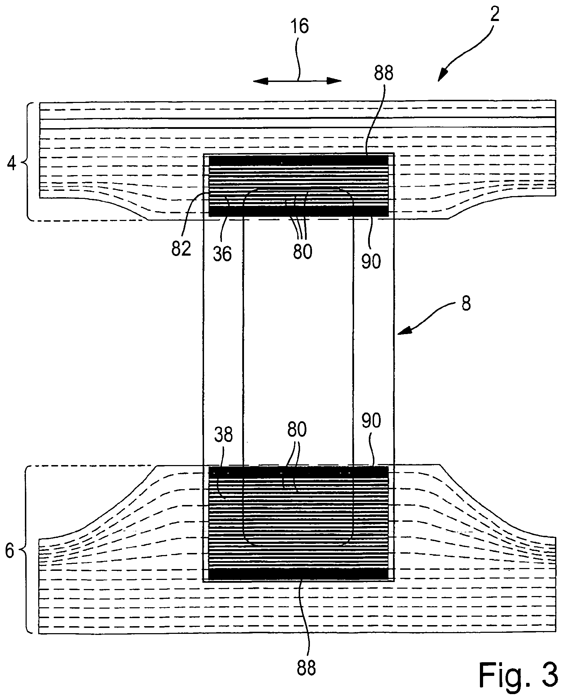

Nachfolgend wird die Fixierung des Schrittabschnitts 8 im vorderen Überlappungsbereich 36 mit dem Bauchabschnitt 4 und im hinteren Überlappungsbereich 38 mit dem Rückenabschnitt 6 beschrieben. Wie anhand der Figuren 3 und 4 ersichtlich, ist hierfür kein vollflächiger Kleberauftrag, sondern eine Vielzahl von im Überlappungsbereich vorgesehener, in Querrichtung 16 erstreckter und parallel zueinander verlaufender Klebestreifen 80 vorgesehen, die durch kleberfreie Streifen 82 voneinander beabstandet sind. Die Klebestreifen 80 erfassen oder überfangen im Wesentlichen den gesamten jeweiligen Überlappungsbereich 36, 38. Im beispielhaft dargestellten, jedoch nicht zwingenden Fall sind in einem in Längsrichtung hüftseitigen Randbereich 84 und in einem in Längsrichtung hüftabgewandten Randbereich 86 des jeweiligen Überlappungsbereichs 36, 38 breitere randseitige Klebestreifen 88 und 90 vorgesehen. Die jeweiligen randseitigen, also hüftzugewandten und hüftfernen Klebestreifen 88, 90 sind mit einer größeren Streifenbreite ausgebildet als die Vielzahl von zwischen ihnen und innen liegenden Klebestreifen 80. Bei einer beispielhaften Ausführungsform beträgt die Breite der randseitigen Klebestreifen 88, 90 quer zu deren Erstreckung 14 mm, die Breite der innen liegenden Klebestreifen 80 2 mm und die Breite der kleberfreien Streifen 82 beträgt 3 mm. Im beispielhaft und bevorzugt dargestellten Fall haben die innen liegenden Klebestreifen 80 vorzugsweise alle dieselbe Breite, und vorzugsweise sind auch die Abstände zwischen ihnen, also die Breite der kleberfreien Streifen 82, gleich. Dessen ungeachtet gelten die eingangs erläuterten Ausführungen zu den Abmessungen und dort beschriebenen Verhältnissen sowie zum Flächengewicht der Kleberbeschichtung bei den Klebestreifen. Auch die Fläche des vorderen und hinteren Überlappungsbereichs 36, 38 bezogen auf die Fläche des Bauchabschnitts 4 bzw. des Rückenabschnitts 6 liegt innerhalb der eingangs erläuterten bevorzugten Grenzen.

Man erkennt des Weiteren aus Figur 3 in Zusammenschau mit Figur 1, dass die zweiten Elastifizierungsmittel 40, 42 im jeweiligen Überlappungsbereich 36, 38 parallel zu den Klebestreifen 80 verlaufen. Auch einige wenige der ersten Elastifizierungsmittel 28 verlaufen im beispielhaft dargestellten Fall im vorderen und hinteren Überlappungsbereich 36, 38 (jedoch auf der körperzugewandten Seite des Schrittabschnitts). Auch die zweiten Elastifizierungsmittel 40, 42 wurden in der Querrichtung 16 durchgehend eingebracht; sie sind im jeweiligen Überlappungsbereich 36, 38 durch eingangs erwähnte Maßnahmen deelastifiziert. Es zeigte sich jedoch, dass die zweiten Elastifizierungsmittel auch im deelastifizierten Zustand - wie eingangs erläutert - erkennbar bleiben. Allerdings werden sie durch die Vielzahl von Klebestreifen 80 kaschiert, und somit wird ihre Erkennbarkeit in erfinderischer Weise reduziert.

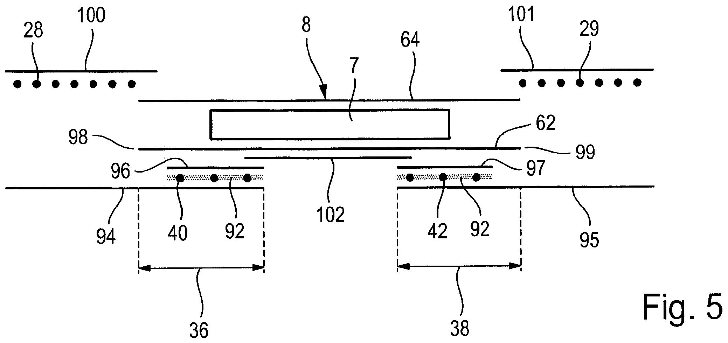

Im bevorzugt dargestellten Fall sind die zweiten Elastifizierungsmittel in einem Leimbett 92 zwischen Chassismateriallagen 94 und 96 bzw. 95 und 97 fixiert (siehe Figur 5). Das Leimbett 92 ist dabei auf eine der Chassismateriallagen 94, 96 bzw. 95, 97 aufgebracht, so dann werden die zweiten Elastifizierungsmittel 40, 42 vorzugsweise endlos auf- bzw. eingelegt und von der weiteren Chassismateriallage überfangen und laminiert. Auf diese Weise werden die zweiten Elastifizierungsmittel 40, 42 fixiert und die Chassismateriallagen 94 und 96 bzw. 95 und 97 flächenhaft durchgehend miteinander gefügt. Bei der körperabgewandten Chassismateriallage 94, 95 handelt es sich um ein atmungsaktives Faservlies, welches der Erstreckung des Bauchabschnitts 4 bzw. Rückenabschnitts 6 entspricht. Bei der Chassismateriallage 96, 97 handelt es sich um ein demgegenüber zurückgesetztes innen liegendes Faservliesmaterial. Es endet in Längsrichtung 9 im bevorzugt dargestellten Fall vor dem Längsende 98, 99 des Schrittabschnitts 8.

Im beispielhaft und bevorzugt dargestellten Fall sind die ersten Elastifizierungsmittel 28, 29 durch Einzelstrangbeleimung zwischen der körperabgewandten Chassismateriallage 94 bzw. 95 und einer weiteren körperzugewandten Chassismateriallage 100, 101 fixiert. Die weitere Chassismateriallage 100, 101 ist wiederum von einem Faservliesmaterial gebildet. Die körperabgewandte und die körperzugewandte Chassismateriallage sind ausschließlich durch die einzelstrangbeleimten ersten Elastifizierungsmittel 28, 29 miteinander verbunden, also nur entlang der Erstreckung dieser ersten Elastifizierungsmittel 28, 29. Die hautfreundlichen Vliesmaterialien sind daher nicht flächenhaft aneinander fixiert, sondern sie können sich voneinander abheben und insbesondere infolge der elastifizierenden Wirkung Fältelungen oder Rüschen bilden. Die körperzugewandte Chassismateriallage 100, 101 erstreckt sich im bevorzugt dargestellten Fall sowohl im Bauchabschnitt 4 als auch im Rückenabschnitt 6 über das jeweilige Längsende 98, 99 des Schrittabschnitts 8 auf dessen körperzugewandter Seite. Es überlappt also diesen Materialübergang und verhindert so eine zu Hautreizungen führende Unstetigkeit.

Man erkennt in Figur 5 des Weiteren, dass das Backsheet 62 des Schrittabschnitts 8 auf seiner körperabgewandten Seite eine Beschichtung 102 aufweist. Diese Beschichtung 102 ist eine Faservliesbeschichtung des im Wesentlichen flüssigkeitsundurchlässigen Backsheets 62. Die Beschichtung 102 erstreckt sich in Längsrichtung 9, jedoch nicht über die gesamte Längserstreckung des Backsheets 62, sondern sie endet verhältnismäßig kurz innerhalb des vorderen und hinteren Überlappungsbereichs 36, 38. Außerhalb des Überlappungsbereichs ist die Beschichtung 102 über die gesamte Erstreckung der körperabgewandten Seite des Backsheets 62 vorgesehen. Die Beschichtung 102 besteht vorzugsweise aus einem Vliesstoff, insbesondere aus einem Spinnvlies, insbesondere aus Polypropylen, insbesondere mit einem Flächengewicht von 10 - 20 g/m², insbesondere von 12 - 17 g/m².

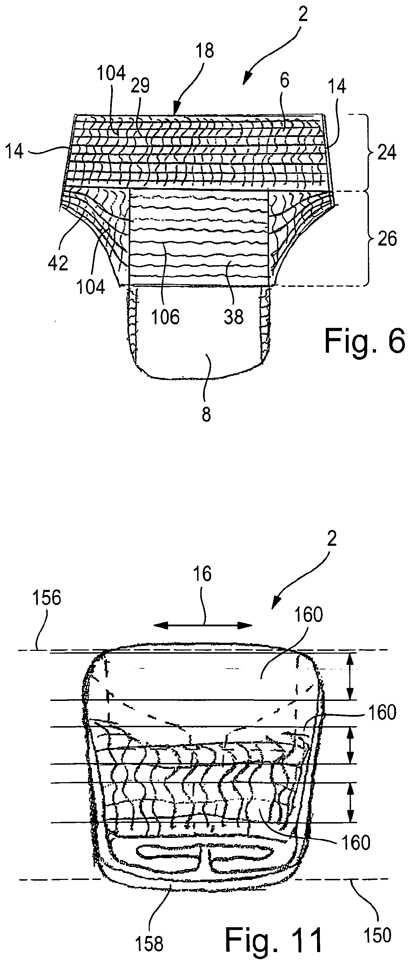

Schließlich zeigt Figur 6 eine schematische Ansicht eines erfindungsgemäßen Inkontinenzartikels im fertig konfigurierten Zustand, in dem der Bauchabschnitt 4 und der Rückenabschnitt 6 durch Ausbildung von Seitennahtbereichen 14 miteinander gefügt sind. Lediglich schematisch angedeutet sind infolge der zusammenziehenden Wirkung der ersten und zweiten Elastifizierungsmittel 28, 29, 40, 42 gebildete Fältelungen oder Rüschen 104, die infolge des Fixierens der Elastifizierungsmittel in vorgedehntem Zustand an den Chassismaterialien (Stretchbondverfahren) gebildet werden. Infolge der Vielzahl von verhältnismäßig feinen Klebestreifen 80 im jeweiligen Überlappungsbereich 36, 38 von Schrittabschnitt 8 und Bauchabschnitt 4 bzw. Rückenabschnitt 6 wird eine visuell und/oder haptisch wahrnehmbare Struktur 106 bei der äußeren Sichtseite des Inkontinenzartikels im jeweiligen Überlappungsbereich 36, 38 gebildet, die hier nur andeutungsweise dargestellt ist. Es wurde erfindungsgemäß festgestellt, dass der streifenförmig aufgebrachte Kleber in die dreidimensional porösen und im Übrigen atmungsaktiv ausgebildeten Faservliesmaterialien, die typischerweise als Chassismaterialien verwendet werden, eindringt und zu einer solchen optisch und/oder haptisch wahrnehmbaren Struktur 106 führt, was sich wie eingangs erläutert als vorteilhaft erweisen kann. Zudem führt die Verbindung von Schrittabschnitt 8 und Bauchabschnitt 4 bzw. Rückenabschnitt 6 durch die Vielzahl von verhältnismäßig schmalen Klebestreifen 80 zu einem sehr wirtschaftlichen Kleberverbrauch, wobei dennoch die erforderlichen Haltekräfte zur sicheren Fügung der drei Komponenten aneinander bereitgestellt werden.

Figur 7 verdeutlicht die Abmessungen, Dimensionen und Verhältnisse eines erfindungsgemäßen Inkontinenzartikels. Man erkennt zunächst, dass die Lage der Quermittelachse 30 die Gesamtlänge des Inkontinenzartikels im flachgelegten Zustand (entsprechend Figur 1) halbiert. Die Quermittelachse 30 bildet auch eine erste in Querrichtung 16 verlaufende Falzachse, um die die Komponenten innerhalb der Herstellungsmaschine gefaltet werden, um die Längsrandabschnitte 10, 12 von Bauchabschnitt 4 und Rückenabschnitt 6 zur Fixierung und Ausbildung von beidseitigen Seitennahtbereichen 14 übereinander zu bringen. Dies findet typischerweise noch unter Führung von endlosen den jeweiligen Bauchabschnitt 4 und Rückenabschnitt 6 bildenden Flachmaterialien, also noch vor der Vereinzelung der Artikel, statt. Man erkennt die Länge L1 zwischen der Quermittelachse 30 und dem jeweiligen Hüftrand 17. Ferner erkennt man die Erstreckung L2 der jeweiligen Seitennaht bzw. des Seitennahtbereichs 14 in Längsrichtung 9, die auch der Länge des jeweiligen Längsrandabschnitts 10 und 12 entspricht. Erfindungsgemäß beträgt das Verhältnis L2/L1 wenigstens 0,42.

Des Weiteren erkennt man den Abstand L4 des äußersten hüftzugewandten ersten Elastifizierungsmittels 28, 29 in Längsrichtung 9 vom innersten schrittzugewandten ersten Elastifizierungsmittel 28, 29. Erfindungsgemäß beträgt das Verhältnis L4/L1 höchstens 0,3.

Man erkennt des Weiteren, dass die ersten Elastifizierungsmittel 28, 29 einen Abstand d₁ voneinander haben, der wenigstens 20 % größer ist als der im Seitennahtbereich 14 bestimmte Abstand der zweiten Elastifizierungsmittel 40, 42 voneinander. Im bevorzugt dargestellten Fall haben die ersten Elastifizierungsmittel 28, 29 alle denselben Abstand d₁ voneinander, der wenigstens 10 mm, insbesondere 10 bis 15 mm beträgt. Das Verhältnis d1/L4 beträgt bevorzugt 0,08 bis 0,25.

Des Weiteren erkennt man L3 als die Erstreckung des Bauchabschnitts 4 und Rückenabschnitts 6 in Längsrichtung 9, welche für den Bauchabschnitt 4 insbesondere 135 - 260 mm und für den Rückenabschnitt 6 insbesondere 200 - 320 mm beträgt.

Weiter dargestellt ist die Erstreckung Q des Bauchabschnitts 4 bzw. des Rückenabschnitts 6 in Querrichtung 16, die in Verhältnisse L2/Q oder L4/Q eingeht.

Die ersten Elastifizierungsmittel 28, 29 haben eine um wenigstens 20 % größere Fadenstärke als die zweiten Elastifizierungsmittel 40, 42. Sie sind zudem mit einer um wenigstens 10 % größeren Vorspannung mit den Chassismateriallagen im Bauchabschnitt 4 und im Rückenabschnitt 6 fixiert als die zweiten Elastifizierungsmittel 40, 42.

Auf die weiteren bevorzugten eingangs gegebenen Abmessungen, Dimensionen und Verhältnisse wird verwiesen.

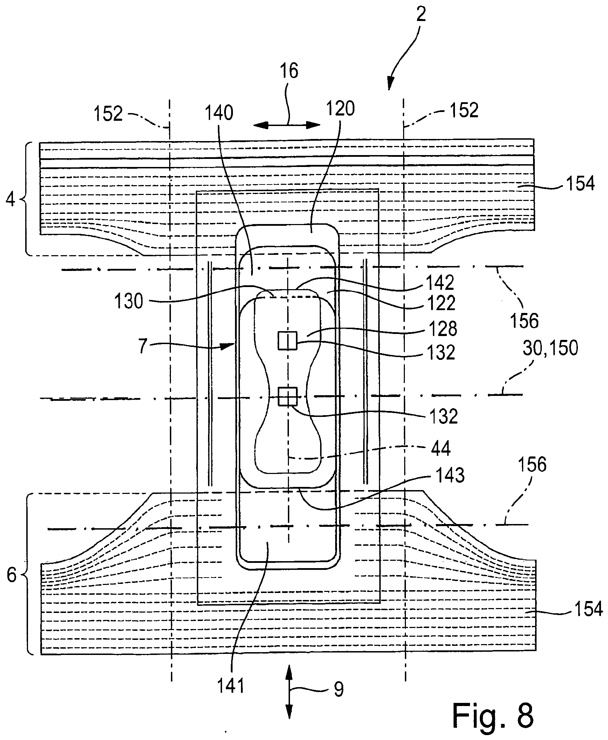

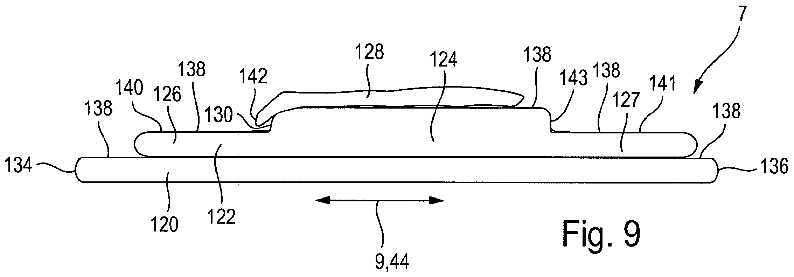

Aus Figuren 8 und 9 ist der Aufbau des Absorptionskörpers 7 in der Draufsicht und in einer Schnittansicht entlang der Längsmittelachse 44 ersichtlich. Der Absorptionskörper 7 umfasst ausgehend von seiner körperabgewandten Seite eine Grundschicht 120 aus zellulosischem Fasermaterial mit einem beispielhaften Flächengewicht von 176 g/m². Je nach exakter flächenhafter Erstreckung enthält die Grundschicht 10 bis 14 g zellulosisches Fasermaterial.

Auf der Grundschicht 120 ist eine Saugkörperschicht 122 abgelegt, die zumindest hinsichtlich des Flächengewichts an Saugkörpermaterial dreidimensional topologisiert ist. Sie weist in einem mittigen Bereich 124 ein höheres Flächengewicht an Saugkörpermaterial auf als in in Längsrichtung 9 vorderen und hinteren Bereichen 126, 127. Das Flächengewicht an zellulosischem Fasermaterial beträgt in dem vorderen und hinteren Bereich 126, 127 der Saugkörperschicht 122 in dem beispielhaft dargestellten Fall 162 g/m² und in dem mittigen Bereich 124 329 g/m². Zusätzlich umfasst die Saugkörperschicht 122 insgesamt etwa 7 g superabsorbierender Polymermaterialien, die homogen gleichförmig in der Saugkörperschicht 122 verteilt angeordnet sind. Die Bereiche 126, 127 und 124 sind in Längsrichtung 9 gegenüber der flächenhaften Erstreckung der Grundschicht 120 zurückgesetzt, wie sich aus Figur 8 ersehen lässt.

Schließlich umfasst der Absorptionskörper 7 eine körperzugewandte im beispielhaft und bevorzugt dargestellten Fall sanduhrförmige Flüssigkeitsaufnahme- und -verteilerschicht 128, die überwiegend auf dem mittleren Bereich 124 der Saugkörperschicht 122 erstreckt ist. Die Flüssigkeitsaufnahme- und -verteilerschicht 128 überragt dabei ein bauchabschnittseitiges Längsende 130 des mittleren Bereichs 124 der Saugkörperschicht 122. Sie umfasst ein Flächengewicht an Fasermaterial, und zwar in Form von intravernetzten Zellulosefasern (curled fiber) von beispielhaft 149 g/m² mit einer Gesamtmasse von entsprechend der beispielhaften Erstreckung ungefähr 2,8 g.

Die Grundschicht 120, die drei Bereiche 124, 126 und 127 der Saugkörperschicht 122 und die körperzugewandte Flüssigkeitsaufnahme- und -verteilerschicht 128 haben über ihre flächenhafte Erstreckung gleichförmiges Flächengewicht an Saugkörpermaterialien.

Das Flächengewicht wird messtechnisch wie eingangs beschrieben ermittelt, indem ein Prüfling von 25 mm x 25 mm betrachtet wird, der durch sämtliche soeben beschriebene Schichten des Absorptionskörpers 7 ausgestanzt wird. Der auszustanzende Flächenbereich 132 (25 mm x 25 mm) wird immer bezüglich der Längsmittelachse 44 zentriert, so wie dies in Figur 8 angedeutet ist. Wenn das Flächengewicht in Längsrichtung 9 weiter vorn oder weiter hinten bestimmt wird, wird der Prüfling in entsprechender Weise bezüglich der Längsmittelachse 44 zentriert.

Man erkennt, dass das Flächengewicht an Saugkörpermaterial so ausgehend von der Quermittelachse 30 entlang der Längsmittelachse 44 in Richtung auf ein bauchabschnittseitige Ende 134 und in Richtung auf ein rückenabschnittseitige Ende 136 des Absorptionskörpers 7 stufenförmig abnimmt. Auf diese Weise werden zwischen den Stufen Plateaus 138 gebildet. Im Bereich dieser Plateaus 138 ist das Flächengewicht an Saugkörpermaterial der darunter liegenden Schichten des Absorptionskörpers 7 vorzugsweise aber nicht notwendigerweise konstant.

Bei der dargestellten bevorzugten Ausführungsform des Inkontinenzartikels ist das Flächengewicht des Absorptionskörpers 7 ausgehend von der Quermittelachse 30 nach vorn und nach hinten im Bereich der Überlappung der körperzugewandten Flüssigkeitsaufnahme- und
-verteilerschicht 128 mit dem mittigen Bereich 124 der Saugkörperschicht 122 im Wesentlichen konstant.

Man erkennt in Figuren 8 und 9 Plateaus 140, 141, die sich an eine Abstufung 142, 143 in Längsrichtung 9 nach vorn bzw. hinten anschließen. Im Bereich dieser Plateaus 140, 141 ist das Flächengewicht des Absorptionskörpers 7 gegenüber dem Flächengewicht im Bereich der Quermittelachse 30 wesentlich reduziert.

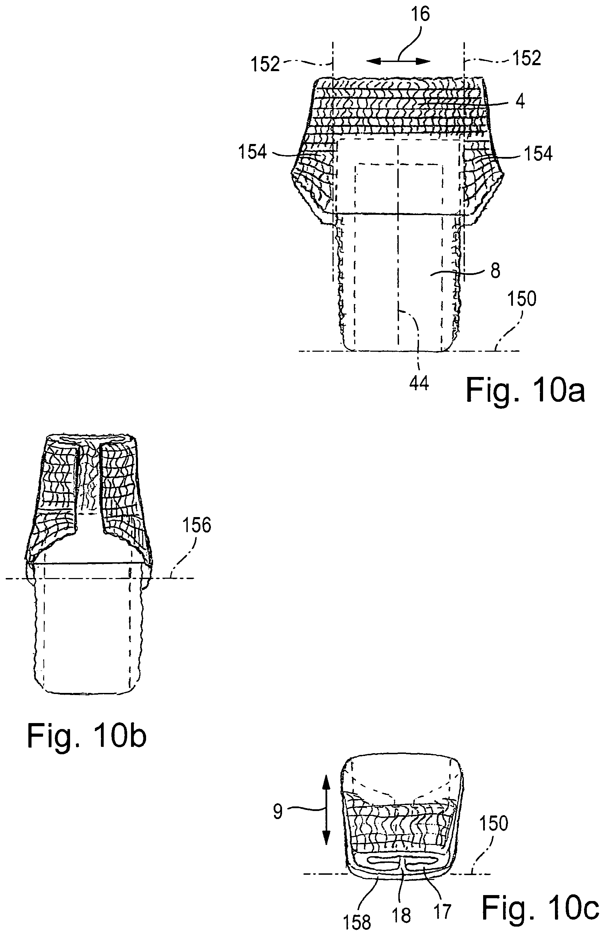

Es wird nun anhand der Figuren 8, 10 und 11 die Faltung des höschenförmigen Inkontinenzartikels für die stapelförmige Anordnung einer Vielzahl von Inkontinenzartikeln in einer Verpackung für die Abgabe in den Handel beschrieben: Wie bereits erwähnt bildet die Quermittelachse 30 eine erste Falzachse 150, um die der Inkontinenzartikel gefaltet wird, so dass Bauchabschnitt 4 und Rückenabschnitt 6 zur Ausbildung von Seitennahtbereichen 14 dauerhaft miteinander gefügt werden können, und zwar durch an sich übliche Fügeverfahren, wie Kleben, Ultraschall, etc.. Des Weiteren sind ungefähr in Längsrichtung 9 erstreckte zweite Falzlinien 152 in Figur 8 lediglich ungefähr angedeutet, da ja die Einfaltung nicht in dem in Figur 8 dargestellten ausgedehnten Zustand erfolgt, sondern nach Fertigstellung des höschenförmigen Inkontinenzartikels in dem in Figur 10a nur schematisch skizzierten Zustand. Ausgehend von diesem in Figur 10a skizzierten Zustand werden beidseitig in Querrichtung 16 seitlich über den Schrittabschnitt 8 überstehende Bereiche 154 des Bauchabschnitts 4 und Rückenabschnitts 6 in Richtung auf die Längsmittelachse 44, und zwar vorzugsweise auf die Außenseite des Bauchabschnitts 4, eingeschlagen, so dass die in Figur 10b ungefähr skizzierte Konfiguration erhalten wird.

Schließlich zeigen die Figuren 8 und 10 eine dritte in Querrichtung 16 verlaufende Falzachse 156, deren Lage bezüglich des Absorptionskörpers 7 aus Figur 8 hervorgeht. Bei weiterer Faltung um diese einzige weitere in Querrichtung 16 erstreckte Falzachse 156 wird die in Figur 10c dargestellte kompakt gefaltete Konfiguration des höschenförmigen Inkontinenzartikels erhalten. Man erkennt, dass der die Hüftöffnung 18 begrenzende Hüftrand 17 in Längsrichtung 9 nicht über die durch die erste Falzachse 150 gebildete äußere Falzkante 158 des Inkontinenzartikels übersteht.

Schließlich verdeutlicht Figur 11, an welchen Stellen die Dicke des insgesamt in die Konfiguration der Figur 10c gefalteten Inkontinenzartikels 2 bestimmt wird. Wie bereits erwähnt, wird der gesamte so gefaltete Inkontinenzartikel 2 über die gesamte Querrichtung 16 mit einem Stanzmesser im Abstand von etwa 10 mm von den Falzkanten bzw. Falzachsen 150 und 156 ausgestanzt, so dass streifenförmige Prüflinge 160 gebildet werden. Anhand dieser sämtliche Lagen des Inkontinenzartikels erfassenden Prüflinge 160 wird dann die Dicke wie eingangs beschrieben ermittelt.

## Patentansprüche

1. Gefalteter Inkontinenzartikel (2) in Höschenform für die Aufnahme von Körperausscheidungen mit einem vorderen Bauchabschnitt (4) und einem hinteren Rückenabschnitt (6), die von separaten und in einer Längsrichtung (9) entlang einer Längsmittelachse (44) voneinander beabstandeten Komponenten gebildet sind, jedoch zur Bildung eines in Quer- oder Hüftumfangsrichtung (16) durchgehenden Bauch- und Rückenbands mit einer in Hüftumfangsrichtung geschlossenen Hüftöffnung (18) an beidseitigen Seitennahtbereichen (14) herstellerseitig miteinander verbunden sind, und mit einem einen Absorptionskörper (7) aufweisenden Schrittabschnitt (8), der sich in der Längsrichtung (9) zwischen Bauchabschnitt (4) und Rückenabschnitt (6) erstreckt und mit seiner körperabgewandten Seite an den Bauchabschnitt (4) und an den Rückenabschnitt (6) in einem jeweiligen Überlappungsbereich (36, 38) unlösbar angefügt ist, wobei der Bauchabschnitt (4), der Rückenabschnitt (6) und der Schrittabschnitt (8) gemeinsam Beinöffnungen (19) des Inkontinenzartikels begrenzen, wobei in dem Bauchabschnitt (4) und dem Rückenabschnitt (6) erste Elastifizierungsmittel (28, 29) vorgesehen sind, die sich in einem Abstand voneinander und parallel zueinander in Quer- oder Hüftumfangsrichtung (16) erstrecken und so den Bauchabschnitt (4) und den Rückenabschnitt (6) flächenhaft elastifizieren, wobei in einem schrittseitigen und den Beinöffnungen (19) zugewandten Bereich (22, 26) des Bauchabschnitts (4) und des Rückenabschnitts (6) zweite Elastifizierungsmittel (40, 42) vorgesehen sind, die sich insbesondere ausgehend von den beiden Seitennahtbereichen (14) in Richtung auf eine Längsmittelachse (44) des Inkontinenzartikels erstrecken und sich bis in den Überlappungsbereich (36, 38) von Schrittabschnitt (8) und Bauchabschnitt (4) bzw. von Schrittabschnitt (8) und Rückenabschnitt (6) erstrecken, wobei sie dort ihrer elastifizierenden Wirkung benommen sein können, insbesondere geschnitten sein können, wobei der Inkontinenzartikel herstellerseitig in gefaltete Konfiguration gebracht ist, wobei eine erste Falzachse (150) durch eine Quermittelachse (30) gebildet ist, **dadurch gekennzeichnet, dass** beidseits des Absorptionskörpers (7) und außerhalb des Absorptionskörpers (7) je eine zweite im wesentlichen in Längsrichtung (9) erstreckte Falzachse (152) vorgesehen ist, um welche beidseitig seitlich über den Schrittabschnitt (8) vorstehende Bereiche (154) des Bauchabschnitts (4) und Rückenabschnitts (6) in Richtung auf die Längsmittelachse (44) eingeschlagen sind, und dass genau eine dritte in Querrichtung (16) erstreckte Falzachse (156) im Bereich des Absorptionskörpers (7) vorgesehen ist, um die der Artikel gefaltet ist, und dass nach der Faltung um die dritte Falzachse (156) der Hüftrand (17) des in Quer- oder Hüftumfangsrichtung durchgehenden Bauch- und Rückenbands in Längsrichtung (9) nicht über die erste Falzachse (150), das heißt nicht über die durch die erste Falzachse (150) gebildete äußere Falzkante (158) des Inkontinenzartikels, übersteht, und dass der Absorptionskörper (7) im Bereich der Quermittelachse (30) ein erstes Flächengewicht an Saugkörpermaterial aufweist und dass ausgehend von der Quermittelachse (30) jeweils entlang der Längsmittelachse (44) in Richtung auf das bauchabschnittseitige Ende (134) des Absorptionskörpers (7) und in Richtung auf das rückenabschnittseitige Ende (135) des Absorptionskörpers (7) das Flächengewicht an Saugkörpermaterial abnimmt, und dass die dritte Falzachse (156) in einem solchen Abstand in Längsrichtung (9) von der Quermittelachse (30) vorgesehen ist, dass der Absorptionskörper (7) dort ein Flächengewicht an Saugkörpermaterial aufweist, das höchstens 80 % des Werts des ersten Flächengewichts im Bereich der Quermittelachse (30) beträgt.

2. Inkontinenzartikel nach Anspruch 1, **dadurch gekennzeichnet, dass** das Flächengewicht an Saugkörpermaterial im Bereich der dritten Falzachse (156) höchstens 70 %, insbesondere höchstens 60 %, insbesondere höchstens 50 %, insbesondere wenigstens 20 %, insbesondere wenigstens 30 % des Werts des ersten Flächengewichts beträgt.

3. Inkontinenzartikel nach Anspruch 1 oder 2, **dadurch gekennzeichnet, dass** das Flächengewicht an Saugkörpermaterial ausgehend von der Quermittelachse (30) entlang der Längsmittelachse (44) in Richtung auf das bauchabschnittseitige Ende (134) des Absorptionskörpers (7) und/oder in Richtung auf das rückenabschnittseitige Ende (135) des Absorptionskörpers (7) stufenförmig abnimmt, so dass abgestufte Plateaus (138, 140) gebildet werden.

4. Inkontinenzartikel nach Anspruch 1, 2 oder 3, **dadurch gekennzeichnet, dass** die Plateaus (138, 140) durch gerade und in Querrichtung (16) verlaufende stufenförmige Übergänge begrenzt sind.

5. Inkontinenzartikel nach einem oder mehreren der vorstehenden Ansprüche, **dadurch gekennzeichnet, dass** der Absorptionskörper (7) gerade und in Längsrichtung erstreckte Längsränder aufweist.

6. Inkontinenzartikel nach einem oder mehreren der vorstehenden Ansprüche, **dadurch gekennzeichnet, dass** der Absorptionskörper (7) in seiner bauchabschnittseitigen Hälfte und/oder in seiner rückenabschnittseitigen Hälfte mehrere Plateaus (138, 140) aufweist, wobei deren Flächengewicht an Saugkörpermaterial ausgehend von der Quermittelachse (30) jeweils entlang einer Längsmittelachse (44) in Richtung auf das bauchabschnittseitige Ende (134) des Absorptionskörpers (7) und in Richtung auf das rückenabschnittseitige Ende (135) des Absorptionskörpers (7) von Plateau (138, 140) zu Plateau (138, 140) abnimmt.

7. Inkontinenzartikel nach einem oder mehreren der vorstehenden Ansprüche, **dadurch gekennzeichnet, dass** der Absorptionskörper (7) eine erste Grundschicht (120) und darauf eine zweite durch Flächengewichtsvariation über ihre Erstreckung dreidimensional topologisierte Saugkörperschicht (122) und vorzugsweise darauf eine, insbesondere sanduhrförmige körperzugewandte Flüssigkeitsaufnahme- und -verteilerschicht (128) aufweist.

8. Inkontinenzartikel nach einem oder mehreren der vorstehenden Ansprüche, **dadurch gekennzeichnet, dass** das erste Flächengewicht des Absorptionskörpers (7) ausgehend von der Quermittelachse (30) jeweils entlang der Längsmittelachse (44) in Richtung auf das bauchabschnittseitige Ende (134) des Absorptionskörpers (7) und in Richtung auf das rückenabschnittseitige Ende (135) des Absorptionskörpers (7) im wesentlichen konstant bleibt über eine Erstreckung von wenigstens 20 %, insbesondere von wenigstens 30 %, insbesondere von höchstens 70 % und weiter insbesondere von höchstens 60 % des Abstands der Quermittelachse (30) vom bauchabschnittseitigen Ende (134) des Absorptionskörpers (7) bzw. vom rückenabschnittseitigen Ende (135) des Absorptionskörpers (7).

9. Inkontinenzartikel nach einem oder mehreren der vorstehenden Ansprüche, **dadurch gekennzeichnet, dass** die Längserstreckung eines Plateaus (140), welches sich an eine Abstufung im bauchabschnittseitigen Teil und/oder im rückenabschnittseitigen Teil des Absorptionskörpers (7) in Längsrichtung (9) anschließt und durch welches sich die dritte Falzachse (156) erstreckt, wenigstens 15 %, insbesondere wenigstens 20 %, insbesondere höchstens 50 %, insbesondere höchstens 40 %, insbesondere höchstens 30 % des Abstands der Quermittelachse (30) vom bauchabschnittseitigen Ende (134) des Absorptionskörpers (7) bzw. vom rückenabschnittseitigen Ende (135) des Absorptionskörpers (7) beträgt.

10. Inkontinenzartikel nach einem oder mehreren der vorstehenden Ansprüche, **dadurch gekennzeichnet, dass** die dritte Falzachse (156) im Überlappungsbereich (38, 36) von Schrittabschnitt (8) und Rückenabschnitt (6) und/oder im Überlappungsbereich von Schrittabschnitt (8) und Bauchabschnitt (4) verläuft.

11. Inkontinenzartikel nach einem oder mehreren der vorstehenden Ansprüche, **dadurch gekennzeichnet, dass** die Dicke des gefalteten Inkontinenzartikels gemessen unter einem Prüfdruck von 20 g/cm² an drei verschiedenen Stellen, nämlich in einem 10 mm von dem der ersten Falzachse (150) zugeordneten Rand beabstandeten Bereich, und in einem 10 mm von dem der dritten Falzachse (156) zugeordneten Rand beabstandeten Bereich und in einem in Längsrichtung (9) dazwischen liegenden Bereich, jeweils um weniger als 6 %, insbesondere um weniger als 5 %, insbesondere um weniger als 4 %, insbesondere um weniger als 3 % von einem arithmetischen Mittelwert der Messungen an den drei Stellen abweicht.

12. Inkontinenzartikel nach einem oder mehreren der vorstehenden Ansprüche, **dadurch gekennzeichnet, dass** die Erstreckung (L2) der jeweiligen Seitennaht in Längsrichtung (9) 100 - 170 mm beträgt und dass das Verhältnis (L2/L1) der Erstreckung (L2) der jeweiligen Seitennaht in Längsrichtung (9) zur Erstreckung (L1) des Inkontinenzartikels zwischen Hüftrand (17) und einer Quermittelachse (30) höchstens 0,42, insbesondere höchstens 0,4, insbesondere höchstens 0,39, insbesondere höchstens 0,38 und weiter insbesondere wenigstens 0,20, weiter insbesondere wenigstens 0,25, weiter insbesondere wenigstens 0,30 beträgt.

13. Inkontinenzartikel nach einem oder mehreren der vorstehenden Ansprüche, **dadurch gekennzeichnet, dass** im Bauchabschnitt (4) und im Rückenabschnitt (6) das Verhältnis (L4/L1) des Abstands (L4) des äußersten hüftzugewandten ersten Elastifizierungsmittels (28, 29) in Längsrichtung (9) vom innersten schrittzugewandten ersten Elastifizierungsmittel (28, 29) zur Erstreckung (L1) des Inkontinenzartikels zwischen Hüftrand (17) und der Quermittelachse (30) höchstens 0,3, insbesondere höchstens 0,29 und insbesondere wenigstens 0,12, insbesondere wenigstens 0,15, insbesondere wenigstens 0,18 beträgt.

14. Inkontinenzartikel nach einem oder mehreren der vorstehenden Ansprüche, **dadurch gekennzeichnet, dass** im Bauchabschnitt (4) und/oder im Rückenabschnitt (6) das Verhältnis (d₁/L4) des Abstands (d₁) der ersten Elastifizierungsmittel (28, 29) in Längsrichtung (9) voneinander zum Abstand (L4) des äußersten hüftzugewandten ersten Elastifizierungsmittels (28, 29) in Längsrichtung (9) vom innersten schrittzugewandten ersten Elastifizierungsmittel (28, 29) zwischen 0,08 und 0,25, insbesondere zwischen 0,09 und 0,20, insbesondere zwischen 0,10 und 0,18 beträgt.

15. Inkontinenzartikel nach einem oder mehreren der vorstehenden Ansprüche, **dadurch gekennzeichnet, dass** die Fadenstärke der ersten Elastifizierungsmittel (28, 29) wenigstens 1000 dtex, insbesondere wenigstens 1100 dtex, insbesondere wenigstens 1200 dtex, insbesondere 1200 - 1500 dtex, insbesondere 1200 - 1400 dtex beträgt und/oder dass die Fadenstärke der zweiten Elastifizierungsmittel (40, 42) 500 - 1100 dtex, insbesondere 600 - 1000 dtex, insbesondere 700 - 900 dtex beträgt.

16. Inkontinenzartikel nach einem oder mehreren der vorstehenden Ansprüche, **dadurch gekennzeichnet, dass** die ersten Elastifizierungsmittel (28, 29) mit einer Vorspannung fixiert sind, welche um den Faktor von wenigstens 1,1, insbesondere wenigstens 1,2, insbesondere wenigstens 1,3 und insbesondere höchstens 2,0, insbesondere höchstens 1,8, insbesondere höchstens 1,6 größer ist als die Vorspannung, mit welcher die zweiten Elastifizierungsmittel (40, 42) fixiert sind.

17. Inkontinenzartikel nach einem oder mehreren der vorstehenden Ansprüche, **dadurch gekennzeichnet, dass** der Schrittabschnitt (8) mittels einer Vielzahl von im Überlappungsbereich (36) von Schrittabschnitt (8) und Bauchabschnitt (4) und im Überlappungsbereich (38) von Schrittabschnitt (8) und Rückenabschnitt (6) vorgesehener, in Querrichtung (16) erstreckter, parallel zueinander verlaufender und voneinander durch kleberfreie Streifen (82) beabstandeter Klebestreifen (80) unlösbar mit dem Bauchabschnitt (4) und mit dem Rückenabschnitt (6) verbunden ist, wobei die Klebestreifen (80) im wesentlichen den gesamten jeweiligen Überlappungsbereich (36, 38) erfassen, und dass die Breite zumindest derjenigen Klebestreifen (80), die bezüglich optionaler randseitiger Klebestreifen (88, 100) innen liegen, quer zu ihrer Erstreckung wenigstens 1 mm bis höchstens 5 mm beträgt, und dass die Breite der kleberfreien Streifen (82) quer zu ihrer Erstreckung wenigstens 1 mm bis höchstens 15 mm beträgt.

## Claims

1. Folded incontinence article (2) in pant form for absorbing bodily excretions with a front stomach section (4) and a rear back section (6), which are formed by separate components which are spaced apart from each other along a longitudinal center axis (44) in a longitudinal direction, however which are connected with each other by the manufacturer for forming a stomach and back band which is continuous in transverse or waist circumference direction (16) and has a waist opening (18) which is closed in the waist circumferential direction, and with a crotch section (8) which has an absorption body (7), which crotch section (8) extends in the longitudinal direction (9) between the stomach section (4) and the back section (6) and is non detachably attached with its body side taring away from the averted side to the stomach section (4) and to the back section (6) in a respective overlapping region (36, 38), wherein the stomach section (4) the back section (6) and the crotch section (8) together form leg openings (19) of the incontinence article, wherein in the stomach section (4) and the back section (6) first elastifying means (28, 29) are provided which extend parallel and spaced apart by a distance to each other in the transverse or waist circumference direction (16) and thereby extensively elastify the stomach section (4) and the back section (6), wherein in a crotch side region (22, 26) of the stomach section (4) and the back section (6) which faces the leg openings (19) second elastifying means (40, 42) are provided which in particular starting from the two lateral seam regions (14) extend in the direction toward a longitudinal center axis (44) of the incontinence article and as far as into the overlapping region (36, 38) of crotch section (8) and stomach section (4) and of crotch section (8) and back section (6), respectively where their elastifying effect may be absent, and they can in particular be cut, wherein the incontinence article is brought into a folded configuration by the manufacturer, wherein a first folding axis (150) is formed by a transverse center axis (30), **characterized in that** on both sides of the absorption body (7) and outside of the absorption body (7) a second folding axis (152) which essentially extends in the longitudinal direction (9) is provided and about which regions (154) of the stomach section (4) and back section (6) which protrude on both sides over the crotch section (8) are folded in the direction of the longitudinal center axis (44) and **in that** exactly one third folding axis (156) which extends in the transverse direction (16) is provided in the region of the absorption body (7) about which the article is folded, and **in that** after the folding about the third folding axis (156) the waist edge (17) of the stomach and back band which is continuous in the transverse or waist circumference direction does not protrude over the first folding axis (150) which means not over the outer folding edge (158) of the incontinence article which is formed by the first folding axis (150), and that the absorption body (7) in the region of the transverse center axis (30) has a first mass per area of absorption body material and that starting from the transverse center axis (30) respectively along the longitudinal center axis (44) in the direction toward the stomach section side end (134) of the absorption body (7) and in direction toward the back section side end (135) of the absorption body (7) the mass per area of absorption body material decreases, and that the third folding axis (156) is provided at a distance to the transverse center axis (30) in the longitudinal direction (9) so that the absorption body (7) at this location has a mass per area of absorption body material which is at most 80% of the value of the first mass per area in the region of the transverse center axis (30).

2. The incontinence article of claim 1, **characterized in that** the mass per area of absorption body material in the region of the third folding axis (150) is at most 70%, in particular at most 60%, in particular at most 50%, in particular at least 20%, in particular at least 30% of the value of the first mass per area.

3. The incontinence article of claim 1 or 2, **characterized in that** the mass per area of absorption body material starting from the transverse center axis (30) along the longitudinal center axis (44) in the direction toward the stomach section side end (134) of the absorption body (7) and/or in the direction toward the back section side end (135) of the absorption body (7) decreases stepwise so that stepped plateaus (138, 140) are formed.

4. The incontinence article according to claim 1, 2 or 3, **characterized in that** the plateaus (138, 140) are delimited by step-shaped straight, transitions extending in the transverse direction (16).

5. The incontinence article according to one or more of the preceding claims, **characterized in that** the absorption body (7) has longitudinal borders that extend straight and in longitudinal direction.

6. The incontinence article according to one or more of the preceding claims, **characterized in that** the absorption body (7) in its stomach section side half and/or in its back section side half has multiple plateaus (138, 140), wherein a mass per area of the plateaus of absorption body material starting from the transverse center axis (30) in each case along a longitudinal center axis (44) in the direction toward the stomach section side end (134) of the absorption body (7) and in the direction toward the back section side end (135) of the absorption body (7) decreases from plateau (18, 140) to plateau (138, 140).

7. The incontinence article according to one or more of the preceding claims, **characterized in that** the absorption body (7) has a first base layer (120) and an absorption body layer (122) arranged thereon, said absorption body layer (122) varying in its mass per area over its extend thereby resulting in a three-dimensional topology over an extent of the absorption body layer, said absorption body preferably having a in particular hourglass-shaped, body-facing liquid-acquisition and -distribution layer (128) arranged on the absorption body layer (122).

8. The incontinence article according to one or more of the preceding claims, **characterized in that** the first mass per area of the absorption body (7) starting from the transverse center axis (30) in each case along the longitudinal center axis (44) in the direction toward the stomach section side end (134) of the absorption body (7) and in the direction toward the back section side end (135) of the absorption body (7) remains essentially constant over an extent of at least 20%, in particular at least 30%, in particular of its most 70% and further in particular of that most 60% of the distance of the transverse center axis (30) to the stomach section side end (134) of the absorption body (7) or to the back section side end (135) of the absorption body (7).

9. The incontinence article according to one or more of the preceding claims, **characterized in that** the longitudinal extension of one of the plateaus (140) which adjoins a step in the stomach section side part and/or in the back section side part of the absorption body (7) in longitudinal direction and through which the third folding axis (156) extends is at least 15%, in particular at least 20%, in particular at most 50%, in particular at most 40%, in particular at most 30% of the distance of the transverse center axis (30) to the stomach section side end (134) of the absorption body (7) or to the back section side end (135) of the absorption body (7).

10. The incontinence article according to one or more of the preceding claims, **characterized in that** the third folding axis (156) extends in the overlapping region (38, 36) of crotch section (8) and back section (6) and/or in the overlapping region of crotch section (8) and stomach section (4).

11. The incontinence article according to one or more of the preceding claims, **characterized in that** the thickness of the folded incontinence article measured under a test pressure of 20 g/cm² at three different sites, namely, in a first region spaced apart by 10 mm from a border associated with the first folding axis (150), and a second region spaced apart by 10 mm from a border associated with the third folding axis (156), and in a region located in the longitudinal direction (9) between the first region and the second region, deviates by less than 6%, in particular by less than 5%, in particular by less than 4%, in particular by less than 3% from an arithmetic mean value of the measurements taken at the three sites.

12. The incontinence article according to one or more of the preceding claims, **characterized in that** the extent (L2) of the respective lateral seam in longitudinal direction (9) is 100 - 170 mm, and that the ratio (L2/L1) between the extent (L2) of the respective lateral seam in the longitudinal direction and the extent (L1) of the incontinence article between the waist border (17) and a transverse center axis (30) is at most 0.42, in particular at most 0.4, in particular at most 0.39, in particular at most 0.38 and further in particular at least 0.20, further in particular at least 0.25, further in particular at least 0.30.

13. The incontinence article according to one or more of the preceding claims, **characterized in that** in the stomach section (4) and in the back section (6) the ratio (L4/L1) between the distance (L4) of the outermost waist-facing first elastifying means (28, 29) in longitudinal direction (9) to the innermost crotch-facing first elastifying means (28, 29) and the extent (L1) of the incontinence article between waist border (17) and the transverse center axis (30) is at most 0.3, in particular at most 0.29 and in particular at least 0.12, in particular at least 0.15, in particular at least 0.18.

14. The incontinence article according to one or more of the preceding claims, **characterized in that** in the stomach section (4) and/or in the back section (6) the ratio (d₁/L4) between the distance (d₁) of the first elastifying means (28, 29) in longitudinal direction (9) to each other and the distance (14) of the outermost waist-facing first elastifying means (28, 29) to the innermost crotch-facing first elastifying means (28, 29) is between 0.08 and 0.25, in particular between 0.09 and 0.20, in particular between 0.10 and 0.18.

15. The incontinence article according to one or more of the preceding claims, **characterized in that** the thread strength of the first elastifying means (28, 29) is at least 1000 dtex, in particular at least 1100 dtex, in particular at least 1200 dtex, in particular 1200 - 1500 dtex, in particular 1200 - 1400 dtex and/or that the thread strength of the second elastifying means (40, 42) is 500 - 1100 dtex, in particular 600 - 1000 dtex, in particular 700 - 900 dtex.

16. The incontinence article according to one or more of the preceding claims, **characterized in that** the first elastifying means (28, 29) are fixed with a pre- tension which is greater by at least the factor 1.1, in particular at least 1.2, in particular at least 1.3 and in particular at most 2.0, in particular at most 1.8, in particular at most 1.6 than the pre-tension with which the second elastifying means (40, 42) are fixed.

17. The incontinence article according to one or more of the preceding claims, **characterized in that** the crotch section (8) is none-detachably connected to the stomach section (4) and to the back section (6) with plural adhesive strips (80) provided in the overlapping region of (36) of crotch section (8) and stomach section (4), and in the overlapping region (38) of crotch section (8) and back section (6), which plural adhesive strips extend parallel to one another in the transverse direction (16) and are separated from each other by adhesive-free strips (82), wherein the adhesive strips (80) occupy essentially the entire respective overlapping region (36, 38), and that the width at least of those adhesive strips (80), that are located inwardly relative to optional border side adhesive strips (88, 100), is 1 mm to at most 5 mm transverse to their extent, and that the width of the adhesive-free strips (82) transverse to their extent is at least 1 mm to at most 15 mm.

## Revendications

1. Article d'incontinence (2) plié, sous forme de culotte, destiné à recueillir les excrétions corporelles, comprenant une portion ventrale avant (4) et une portion dorsale arrière (6) qui sont formées par des composants séparés et espacés l'un de l'autre dans une direction longitudinale (9) suivant un axe médian longitudinal (44), mais qui, pour la formation d'une bande ventrale et dorsale continue dans le sens transversal ou dans le sens du tour de hanches (16), avec une ouverture de hanche (18) fermée dans le sens du tour de hanches, sont reliées entre elles par le fabricant au niveau de zones de couture latérale (14) situées de part et d'autre, et comprenant une portion d'entrejambe (8) présentant un corps absorbant (7), qui s'étend dans la direction longitudinale (9) entre la portion ventrale (4) et la portion dorsale (6) et qui est rapportée de manière inamovible, par sa face montrant dans la direction opposée au corps, sur la portion ventrale (4) et sur la portion dorsale (6) dans une zone de chevauchement (36, 38) respective, dans lequel ladite portion ventrale (4), ladite portion dorsale (6) et ladite portion d'entrejambe (8) délimitent ensemble des ouvertures de jambe (19) de l'article d'incontinence, dans lequel de premiers moyens d'élastification (28, 29) sont prévus dans la portion ventrale (4) et dans la portion dorsale (6), qui s'étendent à distance les uns des autres et parallèlement entre eux dans le sens transversal ou dans le sens du tour de hanches (16) et qui élastifient ainsi en nappe lesdites portions ventrale (4) et dorsale (6), dans lequel de seconds moyens d'élastification (40, 42) sont prévus dans une zone (22, 26) de la portion ventrale (4) et de la portion dorsale (6), qui est située côté entrejambe et montre vers les ouvertures de jambe (19), seconds moyens d'élastification qui s'étendent en particulier à partir des deux zones de couture latérale (14) en direction d'un axe médian longitudinal (44) de l'article d'incontinence et s'étendent jusque dans la zone de chevauchement (36, 38) des portions d'entrejambe (8) et ventrale (4) ou bien des portions d'entrejambe (8) et dorsale (6), ils y pouvant être dépourvus de leur effet élastifiant, en particulier être coupés, dans lequel l'article d'incontinence est mis en configuration pliée par le fabricant, dans lequel un premier axe de pliage (150) est formé par un axe médian transversal (30), **caractérisé par le fait que** de part et d'autre du corps absorbant (7) et hors du corps absorbant (7) est prévu respectivement un deuxième axe de pliage (152) qui s'étend pour l'essentiel dans la direction longitudinale (9) et autour duquel des zones (154) des portions ventrale (4) et dorsale (6), qui dépassent latéralement de part et d'autre ladite portion d'entrejambe (8), sont pliées vers l'intérieur en direction de l'axe médian longitudinal (44), et qu'exactement un troisième axe de pliage (156) s'étendant dans le sens transversal (16) est prévu au niveau du corps absorbant (7), autour duquel l'article est plié, et que, après le pliage autour dudit troisième axe de pliage (156), le bord de hanche (17) de ladite bande ventrale et dorsale continue dans le sens transversal ou dans le sens du tour de hanches ne dépasse pas, dans la direction longitudinale (9), le premier axe de pliage (150), c'est-à-dire ne dépasse pas l'arête de pliage (158) extérieure de l'article d'incontinence formée par le premier axe de pliage (150), et que le corps absorbant (7) présente un premier grammage en matière de corps absorbant au niveau de l'axe médian transversal (30), et que, à partir de l'axe médian transversal (30), respectivement le long de l'axe médian longitudinal (44) en direction de l'extrémité côté portion ventrale (134) du corps absorbant (7) et en direction de l'extrémité côté portion dorsale (135) du corps absorbant (7), le grammage en matière de corps absorbant diminue, et que ledit troisième axe de pliage (156) est prévu à une telle distance de l'axe médian transversal (30), dans la direction longitudinale (9), que le corps absorbant (7) y présente un grammage en matière de corps absorbant qui fait 80 % tout au plus de la valeur du premier grammage au niveau de l'axe médian transversal (30).

2. Article d'incontinence selon la revendication 1, **caractérisé par le fait que** le grammage en matière de corps absorbant au niveau du troisième axe de pliage (156) est de 70 % tout au plus, en particulier de 60 % tout au plus, en particulier de 50 % tout au plus, en particulier de 20 % au moins, en particulier de 30 % au moins, de la valeur du premier grammage.

3. Article d'incontinence selon la revendication 1 ou 2, **caractérisé par le fait que**, à partir de l'axe médian transversal (30), le long de l'axe médian longitudinal (44) en direction de l'extrémité côté portion ventrale (134) du corps absorbant (7) et/ou en direction de l'extrémité côté portion dorsale (135) du corps absorbant (7), le grammage en matière de corps absorbant diminue par paliers de manière à ce que des plateaux étagés (138, 140) soient formés.

4. Article d'incontinence selon la revendication 1, 2 ou 3, **caractérisé par le fait que** lesdits plateaux (138, 140) sont limités par des transitions en gradin droites et s'étendant dans le sens transversal (16).

5. Article d'incontinence selon l'une ou plusieurs des revendications précédentes, **caractérisé par le fait que** le corps absorbant (7) présente des bords longitudinaux droits et s'étendant dans la direction longitudinale.

6. Article d'incontinence selon l'une ou plusieurs des revendications précédentes, **caractérisé par le fait que** le corps absorbant (7) présente une pluralité de plateaux (138, 140) dans sa moitié côté portion ventrale et/ou dans sa moitié côté portion dorsale, dans lequel leur grammage en matière de corps absorbant diminue d'un plateau (138, 140) à l'autre (138, 140) à partir de l'axe médian transversal (30), respectivement le long d'un axe médian longitudinal (44) en direction de l'extrémité côté portion ventrale (134) du corps absorbant (7) et en direction de l'extrémité côté portion dorsale (135) du corps absorbant (7).

7. Article d'incontinence selon l'une ou plusieurs des revendications précédentes, **caractérisé par le fait que** le corps absorbant (7) présente une première couche de base (120) et, sur celle-ci, une deuxième couche de corps absorbant (122) ayant une topologie tridimensionnelle par une variation du grammage sur son extension et, de préférence, sur celle-ci, une couche d'absorption et de distribution de liquide (128) montrant vers le corps, en particulier en forme de sablier.

8. Article d'incontinence selon l'une ou plusieurs des revendications précédentes, **caractérisé par le fait que**, à partir de l'axe médian transversal (30), respectivement le long de l'axe médian longitudinal (44) en direction de l'extrémité côté portion ventrale (134) du corps absorbant (7) et en direction de l'extrémité côté portion dorsale (135) du corps absorbant (7), le premier grammage du corps absorbant (7) reste pour l'essentiel constant sur une extension d'au moins 20 %, en particulier d'au moins 30 %, en particulier de 70 % tout au plus et plus particulièrement de 60 % tout au plus, de la distance séparant l'axe médian transversal (30) et l'extrémité côté portion ventrale (134) du corps absorbant (7) ou bien l'extrémité côté portion dorsale (135) du corps absorbant (7).

9. Article d'incontinence selon l'une ou plusieurs des revendications précédentes, **caractérisé par le fait que** l'extension longitudinale d'un plateau (140) qui suit un étage dans la partie côté portion ventrale et/ou dans la partie côté portion dorsale du corps absorbant (7) dans la direction longitudinale (9) et à travers lequel s'étend ledit troisième axe de pliage (156), fait au moins 15 %, en particulier au moins 20 %, en particulier 50 % tout au plus, en particulier 40 % tout au plus, en particulier 30 % tout au plus, de la distance séparant l'axe médian transversal (30) et l'extrémité côté portion ventrale (134) du corps absorbant (7) ou bien l'extrémité côté portion dorsale (135) du corps absorbant (7).

10. Article d'incontinence selon l'une ou plusieurs des revendications précédentes, **caractérisé par le fait que** le troisième axe de pliage (156) s'étend dans la zone de chevauchement (36, 38) des portions d'entrejambe (8) et dorsale (6) et/ou dans la zone de chevauchement des portions d'entrejambe (8) et ventrale (4).

11. Article d'incontinence selon l'une ou plusieurs des revendications précédentes, **caractérisé par le fait que** l'épaisseur de l'article d'incontinence plié, mesurée à une pression d'essai de 20 g/cm² sur trois points différents, à savoir dans une zone située à une distance de 10 mm du bord associé au premier axe de pliage (150) et dans une zone située à une distance de 10 mm du bord associé au troisième axe de pliage (156) et dans une zone située entre celles-ci dans la direction longitudinale (9), diffère de moins de 6 %, en particulier de moins de 5 %, en particulier de moins de 4 %, en particulier de moins de 3 %, d'une valeur moyenne arithmétique des mesures réalisées sur les trois points.

12. Article d'incontinence selon l'une ou plusieurs des revendications précédentes, **caractérisé par le fait que** l'extension (L2) de la couture latérale respective dans la direction longitudinale (9) est comprise entre 100 et 170 mm et que le rapport (L2/L1) de l'extension (L2) de la couture latérale respective dans la direction longitudinale (9) à l'extension (L1) de l'article d'incontinence entre le bord de hanche (17) et un axe médian transversal (30) et de 0,42 tout au plus, en particulier de 0,4 tout au plus, en particulier de 0,39 tout au plus, en particulier de 0,38 tout au plus et plus particulièrement d'au moins 0,20, plus particulièrement d'au moins 0,25, plus particulièrement d'au moins 0,30.

13. Article d'incontinence selon l'une ou plusieurs des revendications précédentes, **caractérisé par le fait que**, dans la portion ventrale (4) et dans la portion dorsale (6), le rapport (L4/L1) de la distance (L4) séparant, dans la direction longitudinale (9), le premier moyen d'élastification (28, 29) situé le plus à l'extérieur et montrant vers la hanche et le premier moyen d'élastification (28, 29) situé le plus à l'intérieur et montrant vers l'entrejambe, à l'extension (L1) de l'article d'incontinence entre le bord de hanche (17) et l'axe médian transversal (30) est de 0,3 tout au plus, en particulier de 0,29 tout au plus et en particulier d'au moins 0,12, en particulier d'au moins 0,15, en particulier d'au moins 0,18.

14. Article d'incontinence selon l'une ou plusieurs des revendications précédentes, **caractérisé par le fait que**, dans la portion ventrale (4) et/ou dans la portion dorsale (6), le rapport (d₁/L4) de la distance (d₁) séparant les premiers moyens d'élastification (28, 29) les uns des autres dans la direction longitudinale (9), à la distance (L4) séparant, dans la direction longitudinale (9), le premier moyen d'élastification (28, 29) situé le plus à l'extérieur et montrant vers la hanche et le premier moyen d'élastification (28, 29) situé le plus à l'intérieur et montrant vers l'entrejambe, est compris entre 0,08 et 0,25, en particulier entre 0,09 et 0,20, en particulier entre 0,10 et 0,18.

15. Article d'incontinence selon l'une ou plusieurs des revendications précédentes, **caractérisé par le fait que** la grosseur du fil des premiers moyens d'élastification (28, 29) est de 1000 dtex au moins, en particulier de 1100 dtex au moins, en particulier de 1200 dtex au moins, en particulier comprise entre 1200 et 1500 dtex, en particulier entre 1200 et 1400 dtex, et/ou que la grosseur du fil des seconds moyens d'élastification (40, 42) est comprise entre 500 et 1100 dtex, en particulier entre 600 et 1000 dtex, en particulier entre 700 et 900 dtex.

16. Article d'incontinence selon l'une ou plusieurs des revendications précédentes, **caractérisé par le fait que** les premiers moyens d'élastification (28, 29) sont fixés avec une précontrainte qui est supérieure du facteur d'au moins 1,1, en particulier d'au moins 1,2, en particulier d'au moins 1,3 et en particulier de 2,0 tout au plus, en particulier de 1,8 tout au plus, en particulier de 1,6 tout au plus, à la précontrainte avec laquelle sont fixés les seconds moyens d'élastification (40, 42).

17. Article d'incontinence selon l'une ou plusieurs des revendications précédentes, **caractérisé par le fait que** ladite portion d'entrejambe (8) est reliée de manière inamovible à la portion ventrale (4) et à la portion dorsale (6) par l'intermédiaire d'une pluralité de rubans adhésifs (80) qui sont prévus dans la zone de chevauchement (36) des portions d'entrejambe (8) et ventrale (4) et dans la zone de chevauchement (38) des portions d'entrejambe (8) et dorsale (6), s'étendent dans le sens transversal (16), s'étendent parallèlement entre eux et sont espacés les uns des autres par des rubans non adhésifs (82), lesdits rubans adhésifs (80) couvrant pour l'essentiel l'ensemble de la zone de chevauchement (36, 38) respective, et que la largeur au moins des rubans adhésifs (80) qui sont situés à l'intérieur par rapport à des rubans adhésifs (88, 100) marginaux optionnels est comprise entre au moins 1 mm et tout au plus 5 mm transversalement à leur extension, et que la largeur des rubans non adhésifs (82) est comprise entre au moins 1 mm et tout au plus 15 mm transversalement à leur extension.
